(19) Europäisches Patentamt
European Patent Office
Office européen des brevets

(11) EP 1 634 619 A1

(12) # EUROPÄISCHE PATENTANMELDUNG

(43) Veröffentlichungstag:
**15.03.2006 Patentblatt 2006/11**

(21) Anmeldenummer: **05013458.4**

(22) Anmeldetag: **22.06.2005**

(51) Int Cl.:
***A61Q 1/02*** *(2006.01)* ***A61Q 1/12*** *(2006.01)*
***A61Q 3/02*** *(2006.01)* ***A61K 8/04*** *(2006.01)*
***A61K 8/81*** *(2006.01)* ***A61K 8/25*** *(2006.01)*
***A61K 8/92*** *(2006.01)*

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **01.07.2004 DE 102004032120**

(71) Anmelder: **Merck Patent GmbH**
**64293 Darmstadt (DE)**

(72) Erfinder:
- **Winkler, Holger, Dr.**
  **64291 Darmstadt (DE)**
- **Horstmann, Stefan, Dr.**
  **64646 Heppenheim (DE)**
- **Schmidt, Christoph, Dr.**
  **65830 Kriftel (DE)**

(54) **Opaleszierende Beugungsfarbmittel für die Kosmetik**

(57) Die Erfindung betrifft die Verwendung von Beugungsfarbmitteln in der Kosmetik, Zubereitungen enthaltend Beugungsfarbmittel sowie Verfahren zur Herstellung der Zubereitungen und deren Verwendung.

EP 1 634 619 A1

## Beschreibung

Die Erfindung betrifft die Verwendung von Beugungsfarbmitteln in der Kosmetik, Zubereitungen enthaltend Beugungsfarbmittel sowie Verfahren zur Herstellung der Zubereitungen und deren Verwendung.

Im Umfeld der dekorativen Kosmetik und auch der Gestaltung von pflegenden oder reinigenden Zubereitungen mit hoher Wertanmutung besteht ständig Bedarf nach neuen Effektmaterialien. Marktübliche Effektfarbmittel sind derzeit neben Farbstoffen beispielsweise Interferenzpigmente.

Jetzt wurde gefunden, dass sich auch Beugungsfarbmittel hervorragend zum Einsatz in der Kosmetik eignen.

Ein erster Gegenstand der vorliegenden Erfindung ist daher die Verwendung von Beugungsfarbmitteln in der Kosmetik.

Dabei werden die Beugungsfarbmittel in einer Erfindungsvariante zu dekorativen Zwecken in der topischen Anwendung eingesetzt. In einer weiteren Variante werden die Beugungsfarbmittel zu dekorativen Zwecken in Zubereitungen zur topischen Anwendung eingesetzt werden. Dabei kann die topische Anwendung auf Haut, Haaren oder Nägeln erfolgen, wobei die Anwendung der Beugungsfarbmittel auf Nägeln erfindungsgemäß besonders bevorzugt ist.

Dabei kann es sich bei bevorzugten Beugungsfarbmitteln um Partikel, insbesondere Plättchen oder Kugeln, Folien oder Aggregate handelt.

Beugungsfarbmittel im Sinne der vorliegenden Erfindung sind Materialien, die dreidimensionale photonische Strukturen aufweisen. Unter dreidimensionalen photonischen Strukturen werden i. a. Systeme verstanden, die eine regelmäßige, dreidimensionale Modulation der Dielektrizitätskonstanten (und dadurch auch des Brechungsindex) aufweisen. Entspricht die periodische Modulationslänge in etwa der Wellenlänge des (sichtbaren) Lichtes, so tritt die Struktur mit dem Licht nach Art eines dreidimensionalen Beugungsgitters in Wechselwirkung, was sich in winkelabhängigen Farberscheinungen äußert.

Daher ist es erfindungsgemäß bevorzugt, wenn die Beugungsfarbmittel in dem für die optischen Eigenschaften wesentlichen Anteil im wesentlichen aus Anordnungen kugelförmiger Teilchen oder Kavitäten mit einer im wesentlichen monodispersen Größenverteilung bestehen.

Ein Beispiel hierfür stellt der in der Natur vorkommende Edelstein Opal dar, der aus einer dichtest gepackten Kugelpackung aus Siliciumdioxidkugeln besteht und dazwischen liegenden Hohlräumen, die mit Luft, Kieselgel oder Wasser gefüllt sind.

Natürliche Edel-Opale sind aus Domänen aufgebaut, bestehend aus monodispersen, dichtgepackten und daher regelmäßig angeordneten Kieselgel-Kugeln mit Durchmessern von 150-400 nm. Das Farbenspiel dieser Opale kommt durch Bragg-artige Streuung des einfallenden Lichtes an den Gitterebenen der kristallartig angeordneten Domänen zustande.

Entsprechende Kugelanordnungen können natürlich auch mit Kugeln aus anderen Materialien erhalten werden. Geeignete Kugeln sind weiter unten in den Absätzen zu Kernen für Kern-Mantel-Partikeln beschrieben. Alle dort als mögliche Kerne beschriebenen Kugelmaterialien eignen sich zur regelmäßigen Anordnung in Beugungsfarbmitteln.

Derartige Kugelaggregate sind insbesondere dann erfindungsgemäß bevorzugt einzusetzende Beugungsfarbmittel, wenn die Aggregate mechanisch stabilisiert sind. Dies kann durch Verknüpfung der Kugeln oder Einbringen einer hochviskosen Matrix zwischen den Kugeln geschehen.

Unter hochviskosen Materialien werden dabei insbesondere zähflüssige, wachsartige oder feste, plastische oder elastische bis spröde Materialien verstanden.

Beispielsweise können Kugelaggregate in eine wachsartige Matrix eingebettet werden. Eine geeignete wachsartige Matrix erzeugen beispielsweise Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren. Zu den besonders geeigneten Substanzen für die wachsartige Matrix zählen Paraffine, Bienenwachs, Candelilawachs, Canaubawachs, Siliconwachse sowie Mikrokristalline wachse (wie z.B. Ozokerit und Ceresin) und insbesondere hautschmelzende Wachse, wie Sheabutter, Lanolin (insbesondere hydrierte Qualitäten) und Myristylmyristat.

Entsprechende Beugungsfarbmittel und ein Verfahren zu deren Herstellung, bei dem in einem ersten Schritt eine dreidimensionale Kugelpackung erzeugt wird und in einem zweiten Schritt die Kugelaggregate mit einer Schmelze eines bei Raumtemperatur wachsartigen Materials getränkt werden, sind weitere Gegenstände der vorliegenden Anmeldung. Beispielsweise können regelmäßige Kugelaggregate durch einfache Sedimentation oder wirtschaftlicher aus einer Dispersion durch Filtration erzeugt werden. Durch bei Wachsen übliche Verarbeitungs- und insbesondere Zerkleinerungsschritte können aus den Wachs-stabilisierten Kugelaggregaten dann beliebige Partikel, wie Plättchen oder Kugeln, erzeugt werden.

Wenn das Wachs so gewählt wird, dass dessen Schmelzpunkt im Bereich zwischen Raumtemperatur und der Temperatur der Hautoberfläche liegt, eignet sich das resultierende Beugungsfarbmittel insbesondere zur Anwendung zu dekorativen Zwecken in Zubereitungen zur topischen Anwendung. Verbleibt die Zubereitung für längere Zeit auf der Haut, so schmilzt das Wachs, die Kugeln verlieren ihre regelmäßige Anordnung und der optische Effekt des Beugungsfarbmittels verschwindet. Zu diesem Zweck besonders geeignete Wachse sind die o.g. hautschmelzenden Wachse.

Derartige Beugungsfarbmittel mit einer lassen sich dann besonders vorteilhaft auch zur Kontrolle der Auftragung von pflegenden oder schützenden Zubereitungen, wie Tagescremes oder Sonnenschutzmitteln oder Insektenschutzzubereitungen, einsetzen.

Ein entsprechendes Verfahren zur Beurteilung der Auftragung von Zubereitungen zur topischen Anwendung, welches dadurch gekennzeichnet ist, dass man Beugungsfarbmittel, welche in die Zubereitung eingearbeitet sind, auf die Haut aufträgt und anhand des sichtbaren Effekts der Beugungsfarbmittel abschätzt, auf welchen Hautpartien bereits ein Auftrag stattgefunden hat und/oder ob genügend Zubereitung aufgetragen worden ist und/oder wann ein erneutes Auftragen erforderlich geworden ist, ist daher ein weiterer Gegenstand der vorliegenden Anmeldung.

Weitere geeignete Stabilisierungsmethoden für Kugelaggregate sind aus der Literatur bekannt. US 4 703 020 beschreibt ein Verfahren zur Herstellung eines dekorativen Materials, das aus amorphen Silica-Kügelchen besteht, die dreidimensional angeordnet sind, wobei sich in den Zwischenräumen zwischen den Kügelchen Zirkoniumoxid oder Zirkoniumhydroxid befindet. Die Kügelchen haben einen Durchmesser von 150-400 nm. Die Herstellung erfolgt dabei in zwei Stufen. In einer ersten Stufe lässt man aus einer wässrigen Suspension Siliciumdioxidkügelchen sedimentieren. Die erhaltene Masse wird dann an der Luft getrocknet und anschließend bei 800 °C kalziniert. Das kalzinierte Material wird in einer zweiten Stufe in die Lösung eines Zirkoniumalkoxides eingebracht, wobei das Alkoxid in die Zwischenräume zwischen den Kernen eindringt und durch Hydrolyse Zirkoniumoxid ausgefällt wird. Dieses Material wird dann anschließend bei 1000-1300 °C kalziniert. Auch dieses Beugungsfarbmittel eignet sich zur erfindungsgemäßen Verwendung.

Aus EP-A-1285031 sind Partikel mit opaleszierendem Effekt bekannt, die eine Teilchengröße im Bereich von 5 μm bis 5000 μm aufweisen, wobei die Teilchen aus monodispersen Kugeln mit einem Durchmesser von 50 nm - 2 μm bei einer Standardabweichung von weniger als 5 % in einer dreidimensionalen, domänenweise dicht gepackten und regelmäßig angeordneten Struktur, die durch eine physikalische oder chemische Modifikation mechanisch stabilisiert ist, bestehen. Beschrieben wird weiter ein Verfahren zur Herstellung von Partikeln, bei denen in einem Schritt a) monodisperse Kugeln mit einem Durchmesser von 50 nm bis 2 μm bei einer Standardabweichung von weniger als 5 % in einem flüssigen Medium suspendiert werden, b) die Suspension auf eine Oberfläche aufgetragen wird, und c) das flüssige Medium entfernt wird. Die Stabilisierung kann durch folgende physikalische und chemische Maßnahmen erreicht werden. Man modifiziert die Oberfläche der Kugeln, um während der Ausbildung der Opalstruktur eine Vernetzung der Kugeln oder eine bessere Haftung der Kugeln aneinander zu erreichen. Zur Suspension der Kugeln in einem flüssigen Medium kann vorzugsweise eine in Wasser hydrolysierbare, Verbindung zugegeben werden, deren Hydrolyseprodukt sich bei der Ausbildung der Opalstruktur auf den Kugeln niederschlägt und eine chemische Verbindung der Kugeln untereinander bewirkt. Im Falle von Kugeln aus Siliciumdioxid wird der Suspension vorzugsweise Tetraethylorthosilikat bei Temperaturen von 50 bis 80 °C zugesetzt, das zu Siliciumdioxid hydrolysiert und zu einer chemischen Verbindung der Kugeln untereinander führt. Alternativ kann auch Siliciumtetrachlorid zur Behandlung der beschichteten Oberfläche eingesetzt werden. Auch durch Zusatz von löslichen Silicaten, beispielsweise Natriumwasserglas und/oder polymerisierbaren löslichen Aluminiumverbindungen in der Suspension, können die erfindungsgemäßen Partikel chemisch stabilisiert werden. Die Stabilisierung kann auch durch eine Behandlung der beschichteten Oberfläche mit löslichen Silicaten erzielt werden. Ebenso können die Partikel physikalisch stabilisiert werden, indem sie in transparente Kunststoffe oder geeignete Lacke eingebettet werden. Dabei ist es für das Einbettungsmaterial wesentlich, dass es transparent ist und einen geeigneten Brechungsindex aufweiset, der zu einer optimalen Brechungsindexdifferenz führt. Für eine leichte Verarbeitung des Einbettungsmaterials, beispielsweise eines Lackes, ist es dabei insbesondere von Vorteil, wenn es niederviskos ist und sein Volumen beim Aushärten nicht oder nur sehr wenig verändert. In einer möglichen Ausführungsform ist das Einbettungsmaterial oder sein Precursor in entsprechendem Volumenanteil schon in der Suspension enthalten. Bei einer weiteren Ausführungsform der Verfestigung der Opalstruktur wird die Oberfläche der Kugeln mit Silanen modifiziert, die dann während der Ausbildung der Opalstruktur durch Wärme oder UV-Strahlung miteinander vernetzt werden. Diese Vernetzung führt ebenfalls zu einer Verfestigung der Opalstruktur. Die Silanisierung von monodispersen Siliciumdioxidkugeln ist in DE 43 16 814 näher beschrieben. Die resultierenden Beugungsfarbmitteln eignen sich zur Verwendung als Pigment in der Kosmetik.

In der EP-A-0 955 323 werden Kern/Schale-Partikel, deren Kern- und Schalenmaterialien ein Zweiphasensystem ausbilden können und die dadurch gekennzeichnet sind, dass das Schalenmaterial verfilmbar ist und die Kerne unter den Bedingungen der Verfilmung der Schale im wesentlichen formbeständig sind, durch das Schalenmaterial nicht oder nur in sehr geringem Ausmaß quellbar sind und eine monodisperse Größenverteilung aufweisen, wobei ein Unterschied zwischen den Brechungsindices des Kernmaterials und des Schalenmaterials von mindestens 0,001 besteht. Ferner wird die Herstellung der Kern/Schale-Partikel sowie ihre Verwendung zur Herstellung von Formkörpern beschrieben. Das Verfahren zur Herstellung eines Formkörpers umfasst dabei die folgenden Schritte: Auftrag der Kern/Schale-Partikel auf ein Substrat geringer Haftfähigkeit, gegebenenfalls Verdunsten lassen oder Abtreiben des eventuell in der aufgetragenen Schicht enthaltenen Lösungs- oder Verdünnungsmittels, Überführung des Schalenmaterials der Kern/Schale-Partikel in eine flüssige, weiche oder visco-elastische MatrixPhase, Orientierung der Kerne der Kern/Schale-Partikel zumindest zu Domänen regelmäßiger Struktur, Aushärtung des Schalenmaterials zur Fixierung der regelmäßigen

Kern-Struktur, Ablösung des ausgehärteten Films vom Substrat und sofern ein Pigment oder ein Pulver hergestellt werden soll, Zerkleinerung des abgelösten Films auf die gewünschte Partikelgröße. Bei diesen in der EP-A-0 955 323 offenbarten Kern-Schale-Partikeln "schwimmt" der Kern in der Schalenmatrix; eine Fernordnung der Kerne bildet sich in der Schmelze nicht aus, sondern lediglich eine Nahordnung der Kerne in Domänen. Dadurch eignen sich diese Partikel nur eingeschränkt zur Verarbeitung mit bei Polymeren üblichen Methoden. Eine Verwendung dieser Aggregate als Beugungsfarbmittel ist ebenfalls ein Gegenstand der vorliegenden Erfindung.

Aus der Patentanmeldung WO 03/25035 sind Formkörper bekannt, die im wesentlichen aus Kern-Mantel-Partikeln, deren Mantel eine Matrix bildet und deren Kern im wesentlichen fest ist und eine im wesentlichen monodisperse Größenverteilung aufweist, bestehen, wobei der Mantel vorzugsweise über eine Zwischenschicht fest mit dem Kern verbunden ist. Dabei unterscheiden sich die Brechungsindices des Kernmaterials und des Mantelmaterials, wodurch besagter optischer Effekt, vorzugsweise eine Opaleszenz entsteht. Gemäß der Patentanmeldung DE 10204338 können in Formkörper solcher Kern-Mantel-Partikel zusätzlich Kontrastmaterialen, wie Pigmente, eingebracht werden. Die eingelagerten Kontrastmaterialien bewirken eine Zunahme von Brillianz, Kontrast und Tiefe der beobachteten Farbeffekte bei diesen Formkörpern. Die Verarbeitung solcher Formkörper zu Pigmenten wird ebenfalls beschrieben. Dabei kann die Herstellung der Pigmente beispielsweise erfolgen indem aus den Kern-Mantel-Partikeln zuerst ein Film hergestellt wird, der ggf. gehärtet werden kann. Anschließend kann der Film in geeigneter Weise durch Schneiden oder Brechen und evtl. anschließendes Mahlen zu Pigmenten geeigneter Größe zerkleinert werden. Dieser Vorgang kann beispielsweise in einem kontinuierlichen Bandverfahren erfolgen. Die Verwendung entsprechender Beugungsfarbmittel gemäß WO 03/25035 bzw. DE 10204338 im wesentlichen aufgebaut aus Kern-Mantel-Partikeln, deren Herstellung und Zusammensetzung gehört ausdrücklich auch zur Offenbarung der vorliegenden Patentanmeldung.

Beugungsfarbmittel im wesentlichen bestehend aus Kern-Mantel-Partikeln, deren Mantel eine Matrix bildet und deren Kern im wesentlichen fest ist und eine im wesentlichen monodisperse Größenverteilung aufweist, wobei ein Unterschied zwischen den Brechungsindices des Kernmaterials und des Mantelmaterials besteht und die Matrix spröde ist, sind in der älteren Deutschen Patentanmeldung mit dem Anmeldeaktenzeichen DE 10357679.7 beschrieben.

Unter einer spröden Matrix wird dabei eine Matrix verstanden, die eine so große mechanische Härte aufweist, dass Filme mit dieser Matrix vermahlen werden können. Insbesondere zeigt eine spröde Matrix und fließt auch bei mechanischer Beanspruchung nicht. Eine spröde Matrix im Sinne der vorliegenden Erfindung bricht bei mechanischer Beanspruchung und eignet sich so in besonderer Weise zur Herstellung von Partikeln durch Vermahlen größerer Einheiten.

Dabei wird die Sprödigkeit der Matrix in einer bevorzugten Ausführungsform der vorliegenden Erfindung dadurch erreicht, dass die Matrix im wesentlichen von vernetzten organischen Polymeren gebildet wird.

In einer ebenfalls bevorzugten Ausführungsform der vorliegenden Erfindung liegt die Glasübergangstemperatur $T_G$ der Matrix oberhalb von 25°C, vorzugsweise oberhalb von 50°C und insbesondere bevorzugt oberhalb von 70°C liegt, so dass unter den üblichen Einsatzbedingungen der Beugungsfarbmittel bei Raumtemperatur oder z. B. durch Sonneneinstrahlung leicht erhöhte Temperatur die Sprödigkeit der Matrix im oben erwähnten Sinn gewährleistet ist. Dabei kann die Glasübergangstemperatur durch geeignete Auswahl von Polymeren als Mantelmaterial der Kern-Mantel-Partikel eingestellt werden. Die gezielte Auswahl bereitet dem Fachmann dabei keinerlei Probleme. Insbesondere bevorzugt handelt es sich bei dem Mantelmaterial um homo- oder copolymeres Poly(cyclohexylmethacrylat), Polystyrol sowie substituierte Polystyrolderivate, wie z. B. Poly(iodstyrol) und Poly(bromstyrol), Polyacrylate und Polymethacrylate mit einer Tg oberhalb der Gebrauchstemperatur, Polyvinylchlorid mit hoher Tg und andere vinylische Polymere, die sich durch Umwandlung aus Polyvinylacetat ergeben, Polyacrylnitril und Styrol-Acrylnitril-Copolymere.

Abgesehen von den diskutierten Aspekten sind diese bevorzugten Beugungsfarbmittel aus Kern-Mantel-Partikeln, wie in WO 03/25035 beschrieben, aufgebaut.

Zur Erzielung des erfindungsgemäßen optischen Effektes ist es wünschenswert, dass die Kern-Mantel-Partikel einen mittleren Teilchendurchmesser im Bereich von etwa 5 nm bis etwa 2000 nm aufweisen. Dabei kann es insbesondere bevorzugt sein, wenn die Kern-Mantel-Partikel einen mittleren Teilchendurchmesser im Bereich von etwa 5 bis 20 nm, vorzugsweise 5 bis 10 nm, aufweisen. In diesem Fall können die Kerne als "Quantum dots" bezeichnet werden; sie zeigen die entsprechenden aus der Literatur bekannten Effekte. Zur Erzielung von Farbeffekten im Bereich des sichtbaren Lichtes ist es von besonderem Vorteil, wenn die Kern-Mantel-Partikel einen mittleren Teilchendurchmesser im Bereich von etwa 40 - 500 nm aufweisen. Insbesondere bevorzugt werden Partikel im Bereich von 80 - 500 nm eingesetzt, da bei Teilchen in diesem Größenordnungsbereich die Reflektionen verschiedener Wellenlängen des sichtbaren Lichtes sich deutlich voneinander unterscheidet und so die für optische Effekte im sichtbaren Bereich besonders wichtige Opaleszenz besonders ausgeprägt in verschiedensten Farben auftritt. In einer Variante der vorliegenden Erfindung ist es jedoch auch bevorzugt, vielfache dieser bevorzugten Teilchengröße einzusetzen, die dann zu Reflexen entsprechend der höheren Ordnungen und damit zu einem breiten Farbenspiel führen.

Unter einem optischen Effekt werden dabei erfindungsgemäß sowohl Effekte im sichtbaren Wellenlängenbereich des Lichtes als beispielsweise auch Effekte im UV- oder Infrarot-Bereich verstanden. In letzter Zeit hat es sich eingebürgert derartige Effekte allgemein als photonische Effekte zu bezeichnen. Alle diese Effekte sind optische Effekte

im Sinne der vorliegenden Erfindung, wobei es sich in einer bevorzugten Ausführungsform bei dem Effekt um eine Opaleszenz im sichtbaren Bereich handelt. Im Sinne einer üblichen Definition des Begriffes handelt es sich bei den erfindungsgemäßen Beugungsfarbmitteln um photonische Kristalle (vgl. Nachrichten aus der Chemie; 49(9) September 2001; S. 1018 - 1025).

Erfindungsgemäß kann es bevorzugt sein, wenn der Kern der Kern-Mantel-Partikel aus einem Material besteht, das entweder nicht oder bei einer Temperatur oberhalb der Fließtemperatur des Mantelmaterials fließfähig wird. Dies kann erreicht werden durch den Einsatz polymerer Materialien mit entsprechend hoher Glasübergangstemperatur ($T_g$), vorzugsweise vernetzter Polymere bzw. durch Einsatz anorganischer Kernmaterialien. Die geeigneten Materialen im einzelnen werden weiter unten beschrieben.

Entscheidend für die Intensität der beobachteten Effekte ist auch die Differenz der Brechungsindices von Kern und Mantel. Erfindungsgemäße Beugungsfarbmittel weisen vorzugsweise eine Differenz zwischen den Brechungsindices des Kernmaterials und des Mantelmaterials von mindestens 0,001, vorzugsweise mindestens 0,01 und insbesondere bevorzugt mindestens 0,1 auf. Sollen die erfindungsgemäßen Beugungsfarbmittel technisch verwertbare photonische Effekte zeigen, so sind Brechungsindexdifferenzen von mindestens 1,5 bevorzugt.

In einer besonderen Ausführungsform der Erfindung sind in die Matrixphase der Beugungsfarbmittel neben den Kernen der Kern-Mantel-Partikel weitere Nanopartikel eingelagert. Diese Partikel werden hinsichtlich ihrer Partikelgröße so ausgewählt, dass sie in die Hohlräume der Kugelpackung aus den Kernen passen und so die Anordnung der Kerne nur wenig verändern. Durch gezielte Auswahl entsprechender Materialien und/oder der Teilchengröße ist es zum einen möglich die optischen Effekte der Beugungsfarbmittel zu verändern, beispielsweise die Intensität zu erhöhen. Zum andern kann durch Einlagerung geeignet "Quantum dots" die Matrix entsprechend funktionalisiert werden. Bevorzugte Materialien sind anorganische Nanopartikel, insbesondere Nanopartikel von Metallen oder von II-VI- bzw. III-V-Halbleitern oder von Materialen, die die magnetischen Eigenschaften der Materialien beeinflussen. Beispiele für bevorzugte Nanopartikel sind Gold, Zinksulfid, Hämatit oder Galliumarsenid.

Der genaue Mechanismus, der zu der gleichmäßigen Orientierung der Kern-Mantel-Partikel in den erfindungsgemäßen Beugungsfarbmitteln führt, ist bislang unbekannt. Es hat sich jedoch gezeigt, dass eine Krafteinwirkung essentiell zur Ausbildung der weitreichenden Ordnung ist. Es wird vermutet, dass die Elastizität des Mantelmaterials unter den Verarbeitungsbedingungen entscheidend für den Ordnungsprozess ist. Die Kettenenden der Mantelpolymere haben im allgemeinen das bestreben Knäuelform anzunehmen. Kommen sich zwei Partikel zu nahe, so werden die Knäuel nach der Modellvorstellung gestaucht und es entstehen abstoßende Kräfte. Da die Mantel-Polymerketten verschiedener Partikel auch miteinander in Wechselwirkung treten, werden die Polymerketten nach dem Modell gestreckt, wenn sich zwei Partikel voneinander entfernen. Durch das Bestreben der Mantel-Polymerketten wieder eine Knäuelform anzunehmen, entsteht eine Kraft, die die Partikel wieder näher zusammen zieht. Nach der Modellvorstellung wird die weitreichende Ordnung der Partikel im Beugungsfarbmittel durch das Wechselspiel dieser Kräfte erzeugt.

Als besonders gut geeignet zur Herstellung erfindungsgemäßer Beugungsfarbmittel haben sich dabei Kern-Mantel-Partikel erwiesen, deren Mantel mit dem Kern über eine Zwischenschicht verbunden ist.

In einer bevorzugten Ausführungsform besteht der Mantel dieser Kern-Mantel-Partikel aus organischen Polymeren, die bevorzugt über eine zumindest teilweise vernetzte Zwischenschicht auf den Kern aufgepfropft sind.

Der Kern kann aus den verschiedensten Materialien bestehen. Wesentlich ist erfindungsgemäß, wie bereits ausgeführt, dass eine Brechungsindexdifferenz zum Mantel besteht und der Kern unter den Verarbeitungsbedingungen fest bleibt.

Weiter ist es in einer Erfindungsvariante insbesondere bevorzugt, wenn der Kern aus einem organischen Polymer, das vorzugsweise vernetzt ist, besteht.

In einer anderen ebenfalls bevorzugten Erfindungsvariante besteht der Kern aus einem anorganischen Material, vorzugsweise einem Metall oder Halbmetall oder einem Metallchalcogenid oder Metallpnictid. Als Chalcogenide werden im Sinne der vorliegenden Erfindung solche Verbindungen bezeichnet, in denen ein Element der 16. Gruppe des Periodensystems der elektronegative Bindungspartner ist; als Pnictide solche, in denen ein Element der 15. Gruppe des Periodensystems der elektronegative Bindungspartner ist.

Bevorzugte Kerne bestehen aus Metallchalcogeniden, vorzugsweise Metalloxiden, oder Metallpnictiden, vorzugsweise Nitriden oder Phosphiden bestehen. Metall im Sinne dieser Begriffe sind dabei alle Elemente, die im Vergleich zu den Gegenionen als elektropositiver Partner auftreten können, wie die klassischen Metalle der Nebengruppen beziehungsweise die Hauptgruppenmetalle der ersten und zweiten Hauptgruppe genauso jedoch auch alle Elemente der dritten Hauptgruppe sowie Silicium, Germanium, Zinn, Blei, Phosphor, Arsen, Antimon und Bismuth. Zu den bevorzugten Metallchalcogeniden und Metallpnictiden gehören insbesondere Silciumdioxid, Aluminiumoxid, Zirkonoxid, Eisenoxide, Titandioxid, Cerdioxid, Galliumnitrid, Bor- und Aluminiumnitrid, Silicium- und Phosphornitrid oder Mischungen davon.

Als Ausgangsmaterial für die Herstellung der erfindungsgemäßen Kern-Mantel-Partikel werden in einer Variante der vorliegenden Erfindung bevorzugt monodisperse Kerne aus Siliciumdioxid eingesetzt, die beispielsweise nach dem in US 4 911 903 beschriebenen Verfahren erhalten werden können. Die Kerne werden dabei durch hydrolytische Polykondensation von Tetraalkoxysilanen in einem wäßrigammoniakalischen Medium hergestellt, wobei man zunächst ein

Sol von Primärteilchen erzeugt und anschließend durch ein kontinuierliches, kontrolliertes Zudosieren von Tetraalkoxysilan die erhaltenen $SiO_2$-Partikel auf die gewünschte Teilchengröße bringt. Mit diesem Verfahren sind monodisperse $SiO_2$-Kerne mit mittleren Teilchendurchmessern zwischen 0,05 und 10 μm bei einer Standardabweichung von 5 % herstellbar.

Weiterhin sind als Ausgangsmaterial $SiO_2$-Kerne bevorzugt, die mit (Halb)Metallen oder nichtabsorbierenden Metalloxiden, wie z.B. $TiO_2$, $ZrO_2$, $ZnO_2$, $SnO_2$ oder $Al_2O_3$, beschichtet sind. Die Herstellung von mit Metalloxiden beschichteter $SiO_2$-Kerne ist beispielsweise in US 5 846 310, DE 198 42 134 und DE 199 29 109 näher beschrieben.

Als Ausgangsmaterial sind auch einsetzbar monodisperse Kerne aus nichtabsorbierenden Metalloxiden wie $TiO_2$, $ZrO_2$, $ZnO_2$, $SnO_2$ oder $Al_2O_3$ oder Metalloxidgemischen. Ihre Herstellung ist beispielsweise in EP 0 644 914 beschrieben. Weiterhin ist das Verfahren gemäß EP 0 216 278 zur Herstellung monodisperser $SiO_2$-Kerne ohne weiteres und mit gleichem Ergebnis auf andere Oxide übertragbar. Zu einem Gemisch aus Alkohol, Wasser und Ammoniak, dessen Temperatur mit einem Thermostaten auf 30 bis 40 °C genau eingestellt wird, werden unter intensiver Durchmischung Tetraethoxysilan, Tetrabutoxytitan, Tetrapropoxyzirkon oder deren Gemische in einem Guss zugegeben und die erhaltene Mischung für weitere 20 Sekunden intensiv gerührt, wobei sich eine Suspension von monodispersen Kerne im Nanometerbereich ausbildet. Nach einer Nachreaktionszeit von 1 bis 2 Stunden werden die Kerne auf die übliche Weise, z.B. durch Zentrifugieren, abgetrennt, gewaschen und getrocknet.

Weiterhin sind als Ausgangsmaterial für die Herstellung der erfindungsgemäßen Kern-Mantel-Partikel auch monodisperse Kerne aus Polymeren geeignet, die Partikel, beispielsweise Metalloxide, eingeschlossen enthalten. Solche Materialien werden beispielsweise von der Firma micro caps Entwicklungs- und Vertriebs GmbH in Rostock angeboten. Nach kundenspezifischen Anforderungen werden Mikroverkapselungen auf der Basis von Polyestern, Polyamiden und natürlichen und modifizierten Kohlenhydraten gefertigt.

Einsetzbar sind weiterhin monodisperse Kerne aus Metalloxiden, die mit organischen Materialien, beispielsweise Silanen, beschichtet sind. Die monodispersen Kerne werden in Alkoholen dispergiert und mit gängigen Organoalkoxysilanen modifiziert. Die Silanisierung sphärischer Oxidpartikel ist auch in DE 43 16 814 beschrieben.

Die Kerne der erfindungsgemäßen Kern-Mantel-Partikel können darüber hinaus auch Farbstoffe enthalten. beispielsweise können sogenannte Nanocolorants, wie sie beispielsweise in WO 99/40123 beschrieben sind, eingesetzt werden. Die Offenbarung der WO 99/40123 wird hiermit ausdrücklich in die Offenbarung der vorliegenden Anmeldung eingeschlossen.

Für die beabsichtigte Verwendung der erfindungsgemäßen Kern/Mantel-Partikel zur Herstellung von Beugungsfarbmitteln ist es wichtig, dass das Mantelmaterial verfilmbar ist, d. h., dass es durch einfache Maßnahmen soweit erweicht, visco-elastisch plastifiziert oder verflüssigt werden kann, dass die Kerne der Kern/Mantel-Partikel zumindest Domänen regelmäßiger Anordnung ausbilden können. Die in der durch Verfilmung der Mantel der Kern/Mantel-Partikel gebildeten Matrix regelmäßig angeordneten Kerne bilden ein Beugungsgitter, das Interferenzerscheinungen hervorruft und dadurch zu sehr interessanten Farbeffekten führt.

Die Materialien von Kern und Mantel können, sofern sie den oben angegebenen Bedingungen genügen, anorganischen, organischen oder auch metallischen Charakter haben oder es können Hybridmaterialien sein.

Im Hinblick auf die Möglichkeit, die erfindungsrelevanten Eigenschaften der Kerne der erfindungsgemäßen Kern/Mantel-Partikel nach Bedarf zu variieren ist es jedoch zweckmäßig, dass die Kerne ein oder mehrere Polymere und/oder Copolymere (Kern-Polymere) enthalten oder dass sie aus solchen Polymeren bestehen.

Vorzugsweise enthalten die Kerne ein einziges Polymer oder Copolymer. Aus dem gleichen Grund ist es zweckmäßig, dass auch die Mantel der erfindungsgemäßen Kern/Mantel-Partikel ein oder mehrere Polymere und/oder Copolymere (Mantel-Polymere; Matrix-Polymere) oder PolymerVorprodukte und gegebenenfalls Hilfs- und Zusatzstoffe enthält, wobei die Zusammensetzung der Mantel so gewählt werden kann, dass sie in nichtquellender Umgebung bei Raumtemperatur im wesentlichen formbeständig und klebfrei ist.

Mit der Verwendung von Polymersubstanzen als Mantelmaterial und ggf. Kernmaterial gewinnt der Fachmann die Freiheit deren relevante Eigenschaften, wie z. B. ihre Zusammensetzung, die Teilchengröße, die mechanischen Daten, den Brechungsindex, die Glasübergangstemperatur, den Schmelzpunkt und das Gewichtsverhältnis von Kern: Mantel und damit auch die anwendungstechnischen Eigenschaften der Kern/Mantel-Partikel festzulegen, die sich letztlich auch auf die Eigenschaften der daraus hergestellten Beugungsfarbmittel auswirken.

Polymere und/oder Copolymere, die in dem Kernmaterial enthalten sein können oder aus denen es besteht, sich hochmolekulare Verbindungen, die der oben für das Kernmaterial gegebenen Spezifikation entsprechen. Geeignet sind sowohl Polymerisate und Copolymerisate polymerisierbarer ungesättigten Monomere als auch Polykondensate und Copolykondensate von Monomeren mit mindestens zwei reaktiven Gruppen, wie z. B. hochmolekulare aliphatische, aliphatisch/aromatische oder vollaromatische Polyester, Polyamide, Polycarbonate, Polyharnstoffe und Polyurethane, aber auch Aminoplast- und Phenoplast-Harze, wie z. B. Melamin/Formaldehyd-, Harnstoff/Formaldehyd- und Phenol/Formaldehyd-Kondensate.

Zur Herstellung von Epoxidharzen die ebenfalls als Kernmaterial geeignet sind, werden üblicherweise Epoxid-Präpolymerisate, die beispielsweise durch Reaktion von Bisphenol A oder anderen Bisphenolen, Resorein, Hydrochinon, Hexandiol oder anderen aromatischen oder aliphatischen Di-oder Polyolen oder Phenol-Formaldehyd-Kondensaten oder deren Mischungen untereinander mit Epichlorhydrin oder anderen Di- oder Polyepoxiden erhalten werden, mit weiteren zur Kondensation befähigten Verbindungen direkt oder in Lösung vermischt und aushärten gelassen.

Zweckmäßigerweise sind die Polymeren des Kernmaterials in einer bevorzugten Erfindungsvariante vernetzte (Co-)Polymere, da diese üblicherweise erst bei hohen Temperaturen ihren Glasübergang zeigen. Diese vernetzten Polymeren können entweder bereits im Verlauf der Polymerisation bzw. Polykondensation oder Copolymerisation bzw. Copolykondensation vernetzt worden sein oder sie können nach Abschluss der eigentlichen (Co-)Polymerisation bzw. (Co-)Polykondensation in einem gesonderten Verfahrensschritt nachvernetzt worden sein.

Für das Mantelmaterial eignen sich, wie für das Kernmaterial, im Prinzip Polymere der oben bereits genannten Klassen, sofern sie so ausgewählt bzw. aufgebaut werden, dass sie der oben für die Mantelpolymeren gegebenen Spezifikation entsprechen.

Polymere, die den Spezifikationen für ein Mantelmaterial genügen, finden sich ebenfalls in den Gruppen der Polymerisate und Copolymerisate polymerisierbarer ungesättigter Monomerer als auch der Polykondensate und Copolykondensate von Monomeren mit mindestens zwei reaktiven Gruppen, wie z. B. der hochmolekularen aliphatischen, aliphatisch/aromatischen oder vollaromatischen Polyester und Polyamide.

Unter Berücksichtigung der obigen Bedingungen für die Eigenschaften der Mantelpolymeren (= Matrixpolymeren) sind für ihre Herstellung im Prinzip ausgewählte Bausteine aus allen Gruppen organischer Filmbildner geeignet. Insbesondere dann, wenn die Matrix des Beugungsfarbmittels aus vernetzten organischen Polymeren gebildet werden soll, kann die Auswahl der Mantelpolymere nahezu beliebig erfolgen. Es muss lediglich gewährleistet werden, dass diese Polymere mit vernetzbaren Gruppen versehen werden können.

Einige weitere Beispiele mögen die breite Palette der für die Herstellung der Mantel geeigneten Polymeren veranschaulichen.

Soll der Mantel vergleichsweise niedrig brechend sein, so eignen sich beispielsweise Polymerisate wie Polyethylen, Polypropylen, Polyethylenoxid, Polyacrylate, Polymethacrylate, Polybutadien, Polymethylmethacrylat, Polytetrafluorethylen, Polyoxymethylen, Polyester, Polyamide, Polyepoxide, Polyurethan, Kautschuk, Polyacrylnitril und Polyisopren.

Soll der Mantel vergleichsweise hochbrechend sein, so eignen sich für den Mantel beispielsweise Polymerisate mit vorzugsweise aromatischer Grundstruktur wie Polystyrol, Polystyrol-Copolymerisate wie z. B. SAN, aromatisch-aliphatische Polyester und Polyamide, aromatische Polysulfone und Polyketone, Polyvinylchlorid, Polyvinylidenchlorid sowie bei geeigneter Auswahl eines hochbrechenden Kernmaterials auch Polyacrylnitril oder Polyurethan.

In einer erfindungsgemäß besonders bevorzugten Ausführungsform von Kern-Mantel-Partikeln besteht der Kern aus vernetztem Polystyrol und der Mantel aus einem Polyacrylat, vorzugsweise Polyethylacrylat und/oder Polymethylmethacrylat, das mit vernetzbaren Monomeren versetzt ist.

Im Hinblick auf die Verarbeitbarkeit der Kern-Mantel-Partikel zu Beugungsfarbmitteln ist es von Vorteil, wenn das Gewichtsverhältnis von Kern zu Mantel im Bereich von 2:1 bis 1:5, vorzugsweise im Bereich von 3:2 bis 1:3 und insbesondere bevorzugt im Bereich von 1:1 bis 2:3 liegt. Generell gilt hier, dass es von Vorteil ist den Mantelanteil zu erhöhen, wenn der Teilchendurchmesser der Kerne ansteigt.

Die erfindungsgemäß einzusetzenden Kern-Mantel-Partikel lassen sich nach verschiedenen Verfahren herstellen. Eine bevorzugte Möglichkeit die Partikel zu erhalten ist ein Verfahren zur Herstellung von Kern-Mantel-Partikeln, durch a) Oberflächenbehandlung monodisperser Kerne, und b) Aufbringen des Mantels aus organischen Polymeren auf die behandelten Kerne.

In einer Verfahrensvariante werden die monodispersen Kerne in einem Schritt a1) durch Emulsionspolymerisation erhalten.

In einer bevorzugten Erfindungsvariante wird auf die Kerne in Schritt a) eine vernetzte polymere Zwischenschicht, vorzugsweise durch Emulsionspolymerisation oder durch ATR-Polymerisation, aufgebracht, die vorzugsweise reaktive Zentren aufweist, an die der Mantel kovalent angebunden werden kann. ATR-Polymerisation steht hier für Atomic Transfer Radicalic Polymerisation, wie sie beispielsweise in K. Matyjaszewski, Practical Atom Transfer Radical Polymerization, Polym. Mater. Sci. Eng. 2001, 84 beschrieben wird. Die Einkapselung anorganischer Materialien mittels ATRP wird beispielsweise in T. Werne, T. E. Patten, Atom Transfer Radical Polymerization from Nanoparticles: A Tool for the Preparation of Well-Defined Hybrid Nanostructures and for Understanding the Chemistry of Controlled/"Living" Radical Polymerization from Surfaces, J. Am. Chem. Soc. 2001, 123, 7497-7505 und WO 00/11043 beschrieben. Die Durchführung sowohl dieser Methode als auch die Durchführung von Emulsionspolymerisationen sind dem Fachmann für Polymerherstellung geläufig und beispielsweise in den o.g. Literaturstellen beschrieben.

Das flüssige Reaktionsmedium, in dem die Polymerisationen oder Copolymerisationen ausgeführt werden können, besteht aus den bei Polymerisationen, insbesondere bei Verfahren der Emulsionspolymerisation, üblicherweise eingesetzten Lösungs-, Dispergier- oder Verdünnungsmitteln. Hierbei wird die Auswahl so getroffen, dass die zur Homogenisierung der Kernpartikel und Mantel-Vorprodukte eingesetzten Emulgatoren eine ausreichende Wirksamkeit entfalten können. Günstig als flüssiges Reaktionsmedium zur Durchführung des erfindungsgemäßen Verfahrens sind

wässrige Medien, insbesondere Wasser.

Zur Auslösung der Polymerisation eignen sich beispielsweise Polymerisationsinitiatoren, die entweder thermisch oder photochemisch zerfallen, Radikale bilden, und so die Polymerisation auslösen. Dabei sind unter den thermisch aktivierbaren Polymerisationsinitiatoren solche bevorzugt, die zwischen 20 und 180 °C, insbesondere zwischen 20 und 80 °C zerfallen. Besonders bevorzugte Polymerisationsinitiatoren sind Peroxide wie Dibenzoylperoxid Di-tert.-Butylperoxid, Perester, Percarbonate, Perketale, Hydroperoxide, aber auch anorganische Peroxide wie $H_2O_2$, Salze der Peroxoschwefelsäure und Peroxo-dischwefelsäure Azoverbindungen, Boralkylverbindungen sowie homolytisch zerfallende Kohlenwasserstoffe. Die Initiatoren und/oder Photoinitiatoren, die je nach den Anforderungen an das polymerisierte Material in Mengen zwischen 0,01 und 15 Gew.-%, bezogen auf die polymerisierbaren Komponenten eingesetzt werden, können einzeln oder, zur Ausnutzung vorteilhafter synergistischer Effekte, in Kombination miteinander angewendet werden. Daneben kommen Redoxsysteme zur Anwendung, wie z.B. Salze der Peroxodischwefelsäure und Peroxoschwefelsäure in Kombination mit niedervalenten Schwefelverbindungen, im speziellen Ammoniumperoxodisulfat in Kombination mit Natriumdithionit.

Auch für die Herstellung von Polykondensationsprodukten sind entsprechende Verfahren beschrieben worden. So ist es möglich, die Ausgangsmaterialien für die Herstellung von Polykondensationsprodukten in inerten Flüssigkeiten zu dispergieren und, vorzugsweise unter Auskreisen niedermolekularer Reaktionsprodukte wie Wasser oder - z. B. bei Einsatz von Dicarbonsäure-di-niederalkylestern zur Herstellung von Polyestern oder Polyamiden - niederen Alkanolen, zu kondensieren.

Polyadditionsprodukte werden analog durch Umsetzung durch Verbindungen erhalten, die mindestens zwei, vorzugsweise drei reaktive Gruppen wie z. B. Epoxid-, Cyanat-, Isocyanat-, oder Isothiocyanatgruppen aufweisen, mit Verbindungen, die komplementäre reaktive Gruppen tragen. So reagieren Isocyanate beispielsweise mit Alkoholen zu Urethanen, mit Aminen zu Harnstoffderivaten, während Epoxide mit diesen Komplementären zu Hydroxyethern bzw. Hydroxyaminen reagieren. Wie die Polykondensationen können auch Polyadditionsreaktionen vorteilhaft in einem inerten Lösungs- oder Dispergiermittel ausgeführt werden.

Es ist auch möglich, aromatische, aliphatische oder gemischte aromatischaliphatische Polymere, z. B. Polyester, Polyurethane, Polyamide, Polyharnstoffe, Polyepoxide oder auch Lösungspolymerisate, in einem Dispergiermittel wie z. B. in Wasser, Alkoholen, Tetrahydrofuran, Kohlenwasserstoffen zu dispergieren oder zu emulgieren (Sekundärdispersion) und in dieser feinen Verteilung nachzukondensieren, zu vernetzen und auszuhärten.

Zur Herstellung der für diese Polymerisations-Polykondensations- oder Polyadditionsverfahren benötigten stabilen Dispersionen werden in der Regel Dispergierhilfsmittel eingesetzt.

Als Dispergierhilfsmittel werden vorzugsweise wasserlösliche hochmolekulare organische Verbindungen mit polaren Gruppen, wie Polyvinylpyrrolidon, Copolymerisate aus Vinylpropionat oder -acetat und Vinypyrrolidon, teilverseifte Copolymeriste aus einem Acrylester und Acrylnitril, Polyvinylalkohole mit unterschiedlichem Restacetat-Gehalt, Zelluloseether, Gelatine, Blockcopolymere, modifizierte Stärke, niedermolekulare, carbon- und/oder sulfonsäuregruppenhaltigen Polymerisate oder Mischungen dieser Stoffe verwendet.

Besonders bevorzugte Schutzkolloide sind Polyvinylalkohole mit einem Restacetat-Gehalt von unter 35, insbesondere 5 bis 39 Mol.-% und/oder Vinylpyrrolidon-/Vinylpropionat-Copolymere mit einem Vinylestergehalt von unter 35, insbesondere 5 bis 30 Gew.-%.

Es können nichtionische oder auch ionische Emulgatoren, gegebenenfalls auch als Mischung, verwendet werden. Bevorzugte Emulgatoren sind gegebenenfalls ethoxyliierte oder propoxylierte längerkettige Alkanole oder Alkylphenole mit unterschiedlichen Ethoxylierungs- bzw. Propoxylierungsgraden (z. B. Addukte mit 0 bis 50 mol Alkylenoxid) bzw. deren neutralisierte, sulfatierte, sulfonierte oder phosphatierte Derivate. Auch neutralisierte Dialkylsulfobernsteinsäureester oder Alkyldiphenyloxiddisulfonate sind besonders gut geeignet.

Besonders vorteilhaft sind Kombinationen dieser Emulgatoren mit den oben genannten Schutzkolloiden, da mit ihnen besonders feinteilige Dispersionen erhalten werden.

Auch spezielle Verfahren zur Herstellung monodisperser Polymerteilchen sind in der Literatur (z. B. R.C. Bakkus, R.C. Williams, J. Appl, Physics 19, S. 1186, (1948) bereits beschrieben worden und können mit Vorteil insbesondere zur Herstellung der Kerne eingesetzt werden. Hierbei ist lediglich darauf zu achten, dass die oben angegebenen Teilchengrößen eingehalten werden. Anzustreben ist weiter eine möglichst hohe Einheitlichkeit der Polymerisate. Insbesondere die Teilchengröße kann dabei über die Auswahl geeigneter Emulgatoren und/oder Schutzkolloide bzw. entsprechender Mengen dieser Verbindungen eingestellt werden.

Durch die Einstellung der Reaktionsbedingungen wie Temperatur, Druck, Reaktionsdauer, Einsatz geeigneter Katalysatorsysteme, die in bekannter Weise den Polymerisationsgrad beeinflussen und die Auswahl der zu ihrer Herstellung eingesetzten Monomeren nach Art und Mengenanteil lassen sich gezielt die gewünschten Eigenschaftskombinationen der benötigten Polymeren einstellen.

Monomere, die zu Polymeren mit hohem Brechungsindex fuhren, sind in der Regel solche, die entweder aromatische Teilstrukturen aufweisen, oder solche, die über Heteroatome mit hoher Ordnungszahl, wie z. B. Halogenatome, insbesondere Brom- oder Jodatome, Schwefel oder Metallionen, verfügen, d. h. über Atome oder Atomgruppierungen, die die Polarisierbarkeit der Polymeren erhöhen.

Polymere mit niedrigem Brechungsindex werden demgemäss aus Monomeren oder Monomerengemischen erhalten, die die genannten Teilstrukturen und/oder Atome hoher Ordnungszahl nicht oder nur in geringem Anteil enthalten.

Eine Übersicht über die Brechungsindices verschiedener gängiger Homopolymerisate findet sich z. B. in Ullmanns Encyklopädie der technischen Chemie, 5. Auflange, Band A21, Seite 169. Beispiele für radikalisch polymerisierbare Monomere, die zu Polymeren mit hohem Brechungsindex führen, sind:

Gruppe a): Styrol, im Phenylkern alkylsubstituierte Styrole, α-Methylstyrol, Mono- und Dichlorstyrol, Vinylnaphthalin, Isopropenylnaphthalin, Isopropenylbiphenyl, Vinylpyridin, Isopropenylpyridin, Vinylcarbazol, Vinylanthracen, N-Benzyl-methacrylamid, p-Hydroxymethacrylsäureanilid.

Gruppe b): Acrylate, die aromatische Seitenketten aufweisen, wie z. B. Phenyl-(meth)acrylat (= abgekürzte Schreibweise für die beiden Verbindungen Phenylacrylat und Phenylmethacrylat), Phenylvinylether, Benzyl-(meth) acrylat, Benzylvinylether, sowie Verbindungen der Formeln

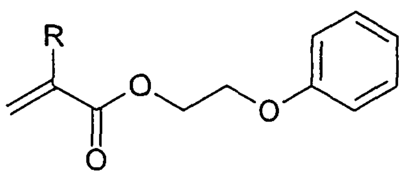

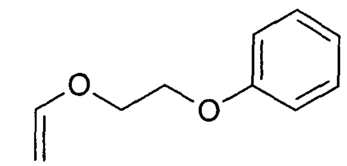

In der obigen und in weiter unten folgenden Formeln sind zur Verbesserung der Übersichtlichkeit und Vereinfachung der Schreibung Kohlenstoffketten nur durch die zwischen den Kohlenstoffatomen bestehenden Bindungen dargestellt. Diese Schreibweise entspricht der Darstellung aromatischer cylischer Verbindungen, wobei z. B. das Benzol durch ein Sechseck mit alternierend einfach und Doppelbindungen dargestellt wird.

Ferner sind solche Verbindungen geeignet, die anstelle von Sauerstoffbrücken Schwefelbrücken enthalten wie z. B.

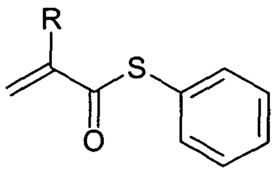

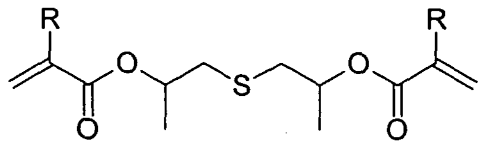

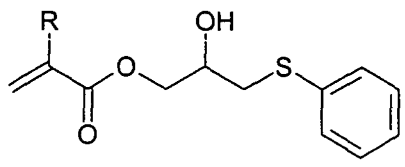

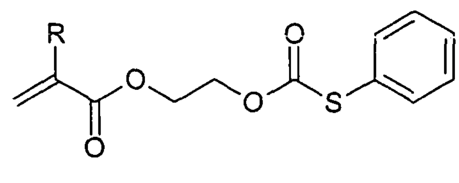

In den obigen Formeln steht R für Wasserstoff oder Methyl. Die Phenylringe dieser Monomeren können weitere Substituenten tragen.

Solche Substituenten sind geeignet, die Eigenschaften der aus diesen Monomeren erzeugten Polymerisate innerhalb gewisser Grenzen zu modifizieren. Sie können daher gezielt benutzt werden, um insbesondere die anwendungstechnisch relevanten Eigenschaften der erfindungsgemäßen Beugungsfarbmittel zu optimieren.

Geeignete Substituenten sind insbesondere Halogen, $NO_2$, Alkyl mit einem bis zwanzig C-Atomen, vorzugsweise Methyl, Alkoxy mit einem bis zwanzig C-Atomen, Carboxyalkyl mit einem bis zwanzig C-Atomen, Carbonyalkyl mit einem bis zwanzig C-Atomen, oder -OCOO-Alkyl mit einem bis zwanzig C-Atomen. Die Alkylketten dieser Reste können ihrerseits gegebenenfalls substituiert sein oder durch zweibindige Heteroatome oder Baugruppen wie z. B. -O-, -S-, -NH-, -COO-, -OCO- oder -OCOO- in nicht benachbarten Stellungen unterbrochen sein.

Gruppe c): Monomere, die über Heteroatome verfügen, wie z. B. Vinylchlorid, Acrylnitril, Methacrylnitril, Acrylsäure, Methacrylsäure, Acrylamid und Methacrylamid oder metallorganische Verbindung wie z. B.

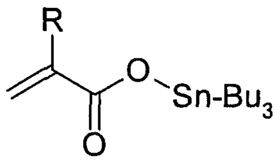

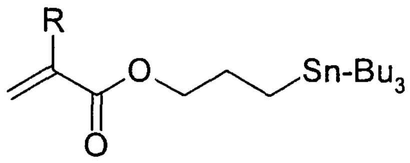

Gruppe d): Eine Erhöhung des Brechungsindex von Polymeren gelingt auch durch Einpolymerisieren Carbonsäuregruppen enthaltender Monomerer und Überführung der so erhaltenen "sauren" Polymeren in die entsprechenden Salze mit Metallen höheren Atomgewichts, wie z. B. vorzugsweise mit K, Ca, Sr, Ba, Zn, Pb, Fe, Ni, Co, Cr, Cu, Mn, Sn oder Cd.

Die oben genannten Monomeren, die einen hohen Beitrag zum Brechungsindex der daraus hergestellten Polymeren leisten, können homopolymerisiert oder untereinander copolymerisiert werden. Sie können auch mit einem gewissen Anteil von Monomeren, die einen geringeren Beitrag zum Brechungsindex leisten, copolymerisiert werden. Solche copolymerisierbaren Monomere mit niedrigerem Brechungsindex-Beitrag sind beispielsweise Acrylate, Methacrylate oder Vinylether oder Vinylester mit rein aliphatischen Resten.

Als vernetzende Mittel zur Herstellung vernetzter Polymerkerne aus radikalisch erzeugten Polymerisaten bzw. zur anschließenden Vernetzung der Matrix aus Matelmaterial können darüberhinaus auch alle bi- oder polyfunktionellen Verbindungen eingesetzt werden, die mit den oben genannten Monomeren copolymerisierbar sind oder die nachträglich mit den Polymeren unter Vernetzung reagieren können.

Im Folgenden sollen Beispiele geeigneter Vernetzer vorgestellt werden, die zur Systematisierung in Gruppen eingeteilt werden:

Gruppe 1: Bisacrylate, Bismethacrylate und Bisvinylether von aromatischen oder aliphatischen di- oder Polydydroxyverbindungen insbesondere von Butandiol (Butandiol-di(meth)acrylat, Butandiol-bis-vinylether), Hexandiol (Hexandiol-di(meth)acrylat, Hexandiol-bis-vinylether), Pentaerythrit, Hydrochinon, Bis-hydroxyphenylmethan, Bishydroxyphenylether, Bis-hydroxymethyl-benzol, Bisphenol A oder mti Ethylenoxidspacern, Propylenoxidspacern oder gemischten Ethlenoxid-Propylenoxidspacern.

Weitere Vernetzer dieser Gruppe sind z. B. Di- oder Polyvinylverbindungen wie Divinybenzol oder auch Methylen-bisacrylamid, Triallylcyanurat, Divinylethylenharnstoff, Trimethylolpropan-tri-(meth)acrylat, Trimethylolpropantricinylether, Pentaerythrit-tetra-(meth)acrylat, Pentaerythrit-tetravinylether, sowie Vernetzer mit zwei oder mehreren verschiedenen reaktiven Enden wie z. B. (Meth)allyl-(meth)acrylate der Formeln

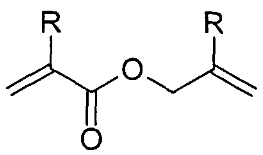

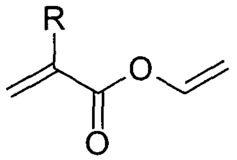

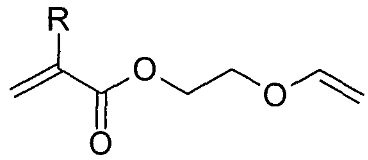

, worin R Wasserstoff oder Methyl bedeutet.

Gruppe 2: Reaktive Vernetzer, die vernetzend, größtenteils aber nachvernetzend wirken, z. B. bei Erwärmung oder Trocknung, und die in die Kern- bzw. Mantelpolymere als Copolymere einpolymerisiert werden.

Beispiele hierfür sind: N-Methylol-(meth)acrylamid, Acrylamidoglycolsäure sowie deren Ether und/oder Ester mit $C_1$ bis $C_6$-Alkoholen, Diacetonacrylamid (DAAM), Glycidylmethacrylat (GMA), Methacryloyloxypropyl-trimethoxysilan (MEMO), Vinyl-trimethoxysilan, m-Isopropenyl-benzylisocyanat (TMI).

Gruppe 3: Carbonsäuregruppen, die durch Copolymerisation ungesättigter Carbonsäuren in das Polymer eingebaut worden sind, werden über mehrwertige Metallionen brückenartig vernetzt. Als ungesättigte Carbonsäuren werden hierzu vorzugsweise Acrylsäure, Methacrylsäure, Maleinsäureandhydrid, Itaconsäure und Furnarsäure eingesetzt. Als Metallionen eignen sich Mg, Ca, Sr, Ba, Zn, Pb, Fe, Ni, Co, Cr, Cu, Mn, Sn, Cd. Besonders bevorzugt sind Ca, Mg und Zn, Ti und Zr.

Gruppe 4: Nachvernetzte Additive. Hierunter versteht man bis- oder höherfunktionalisierte Additive, die mit dem Polymer (durch Additions- oder vorzugsweise Kondensationsreaktionen) irreversibel unter Ausbildung eines Netzwerks reagieren. Beispiele hierfür sind Verbindungen, die pro Molekül mindestens zwei der folgenden reaktiven Gruppen aufweisen: Epoxid-, Aziridin-, Isocyanat-Säurechlorid-, Carbodiimid- oder Carbonylgruppen, ferner z. B. 3,4-Dihydroxy-imidazolinon und dessen Derivate (Fixapret-Marken der BASF).

Wie bereits oben dargelegt, benötigen Nachvernetzer mit reaktiven Gruppen wie z. B. Epoxid- und Isocyanatgruppen komplementäre reaktive Gruppen im zu vernetzenden Polymer. So reagieren Isocyanate beispielsweise mit Alkoholen zu Urethanen, mit Aminen zu Harnstoffderivaten, während Epoxide mit diesen komplementären Gruppen zu Hydroxyethern bzw. Hydroxyaminen reagieren.

Unter Nachvernetzung wird auch die photochemische Aushärtung, eine oxydative oder eine luft- oder feuchtigkeitsinduzierte Aushärtung der Systeme verstanden.

Die oben angegebenen Monomeren und Vernetzer können beliebig und zielgerichtet in der Weise miteinander kombiniert und (co-)polymerisiert werden, dass ein gegebenenfalls vernetztes (Co-)polymerisat mit dem gewünschten Brechungsindex und den erforderlichen Stabilitätskriterien und mechanischen Eigenschaften erhalten wird.

Es ist auch möglich, weitere gängige Monomere, z. B. Acrylate, Methacrylate, Vinylester, Butadien, Ethylen oder Styrol, zusätzlich zu copolymerisieren, um beispielsweise die Glastemperatur oder die mechanischen Eigenschaften der Kern- und/oder Mantelpolymeren nach Bedarf einzustellen.

Erfindungsgemäß ebenfalls bevorzugt ist es, wenn das Aufbringen des Mantels aus organischen Polymeren durch Aufpfropfung, vorzugsweise durch Emulsionspolymerisation oder ATR-Polymerisation erfolgt. Dabei lassen sich die oben beschriebenen Methoden und Monomere entsprechend einsetzen.

Insbesondere beim Einsatz anorganischer Kerne kann es auch bevorzugt sein, dass der Kern vor der Aufpolymerisation des Mantels einer Vorbehandlung unterzogen wird, die ein Anbinden des Mantels ermöglicht. Dies kann üblicherweise in einer chemischen Funktionalisierung der Partikeloberfläche bestehen, wie sie für die verschiedensten anorganischen Materialen aus der Literatur bekannt ist. Insbesondere bevorzugt kann es dabei sein, auf der Oberfläche solche chemischen Funktionen anzubringen, die als aktives Kettenende eine Aufpfropfung der Mantelpolymere ermöglichen. Hier sind als Beispiele insbesondere endständige Doppelbindungen, Epoxy-Funktionen sowie Polykondensierbare Gruppen zu nennen. Die Funktionalisierung von Hydroxygruppentragenden Oberflächen mit Polymeren ist beispielsweise aus EP-A-337 144 bekannt. Weitere Methoden zur Modifizierung von Partikeloberflächen sind dem Fachmann wohl bekannt und beispielsweise in verschiedenen Lehrbüchern, wie Unger, K.K., Porous Silica, Elsevier Scientific Publishing Company (1979) beschrieben.

In einer ebenfalls bevorzugten Ausführungsform der vorliegenden Erfindung enthalten die Beugungsfarbmittel mindestens ein Kontrastmaterial. Die Kontrastmaterialien bewirken eine Zunahme von Brillianz, Kontrast und Tiefe der beobachteten Farbeffekte bei den erfindungsgemäßen Beugungsfarbmitteln. Unter Kontrastmaterialien werden dabei erfindungsgemäß alle Materialien verstanden, die eine solche Verstärkung des optischen Effektes bewirken. Üblicherweise handelt es sich bei diesen Kontrastmaterialien um Pigmente.

Dabei wird unter Pigmenten im Sinne der vorliegenden Erfindung jede feste Substanz verstanden, die im sichtbaren Wellenlängenbereich des Lichtes einen optischen Effekt zeigt. Dabei werden erfindungsgemäß insbesondere solche Substanzen als Pigmente bezeichnet, die der Definition von Pigmenten nach DIN 55943 bzw. DIN 55945 entsprechen. Gemäß dieser Definition handelt es sich bei einem Pigment um ein in Anwendungsmedium praktisch unlösliches, anorganisches oder organisches, buntes oder unbuntes Farbmittel. Dabei können erfindungsgemäß sowohl anorganische als organische Pigmente eingesetzt werden.

Nach ihrer physikalischen Funktionsweise lassen sich Pigmente in Absorptionspigmente und Glanzpigmente einteilen. Bei Absorptionspigmenten handelt es sich um solche Pigmente, die zumindest einen Teil des sichtbaren Lichtes absorbieren und daher einen Farbeindruck hervorrufen und im Extremfall schwarz erscheinen. Glanzpigmente sind nach DIN 55943 beziehungsweise DIN 55944 solche Pigmente bei denen durch gerichtete Reflexion an überwiegend flächig ausgebildeten und ausgerichteten metallischen oder stark lichtbrechenden Pigmentteilchen Glanz-Effekte entstehen. Als Interferenzpigmente werden entsprechend dieser Normen solche Glanzpigmente bezeichnet, deren farbgebende Wirkung ganz oder vorwiegend auf dem Phänomen der Interferenz beruht. Dies sind insbesondere so genannte Perlmutter-Pigmente oder feuergefärbte Metallbronzen. Von wirtschaftlicher Bedeutung unter den Interferenzpigmenten sind insbesondere auch die Perlglanzpigmente, die aus farblosen, transparenten und hoch lichtbrechenden Plättchen bestehen. Sie erzeugten nach Orientierung in einer Matrix einen weichen Glanzeffekt, der als Perlglanz bezeichnet wird. Beispiele für Peilglanzpigmente sind Guanin-haltiges Fischsilber, Pigmente auf Basis von Bleicarbonaten, Bismuthoxidchlorid oder Titandioxid-Glimmer. Insbesondere die Titandioxid-Glimmer, die sich durch mechanische, chemische und thermische Stabilität auszeichnen, werden häufig zu dekorativen Zwecken eingesetzt.

Erfindungsgemäß können sowohl Absorptions- als auch Glanz-Pigmente eingesetzt werden, wobei insbesondere auch Interferenzpigmente eingesetzt werden können. Es hat sich gezeigt, dass insbesondere zur Steigerung der Intensität der optischen Effekte die Verwendung von Absorptionspigmenten bevorzugt ist. Dabei können sowohl Weiß- als auch Farb- oder Schwarzpigmente eingesetzt werden, wobei die Bezeichnung Farbpigmente alle Pigmente meint, die einen anderen Farbeindruck als weiß oder schwarz ergeben, wie beispielsweise Heliogen™ Blau K 6850 (Fa. BASF, Cu-phthalocyanin-Pigment), Heliogen™ Grün K 8730 (Fa. BASF, Cu-phthalocyanin-Pigment), Bayferrox™ 105 M (Fa. Bayer, eisenoxid-basiertes Rotpigment) oder Chromoxidgrün GN-M (Fa. Bayer, chromoxid-basiertes Grünpigment). Aufgrund der erzielten Farbeffekte wiederum bevorzugt sind unter den Absorptionspigmenten die Schwarzpigmente.

Beispielsweise sind hier pigmentärer Russ (z.B. die Carbon Black-Produktlinie der Firma Degussa (insbesondere Purex™ LS 35 bzw. Corax™ N 115)) sowie Eisenoxidschwarz, Manganschwarz sowie Cobaltschwarz und Antimonschwarz zu nennen. Auch schwarze Glimmer-Qualitäten können vorteilhaft als Schwarz-Pigment eingesetzt werden (z. B. Iriodin™ 600, Fa. Merck; Eisenoxidbeschichteter Glimmer).

Es hat sich gezeigt, dass es erfindungsgemäß von Vorteil ist, wenn die Teilchengröße des mindestens einen Kontrastmaterials mindestens doppelt so groß ist wie die Teilchengröße des Kernmaterials. Sind die Teilchen des Kontrastmaterials kleiner, so werden nur unzureichende optische Effekte erzielt. Es wird vermutet, dass kleinere Teilchen, die Ausordnung der Kerne in der Matrix stören und eine Veränderung der sich bildender, Gitter bewirken. Die erfindungsgemäß bevorzugt eingesetzten Teilchen von mindestens doppelter Größe der Kerne wechselwirken mit dem aus den Kern gebildeten Gitter nur lokal. Elektronenmikroskopische Aufnahmen belegen, dass die eingelagerten Partikel das Gitter aus Kernteilchen nicht oder nur wenig stören. Dabei ist mit der Teilchengröße der Kontrastmaterialien, die als Pigmente häufig auch plättchenförmig sind, die jeweils größte Ausdehnung der Teilchen gemeint. Wenn plättchenförmige Pigmente eine Dicke im Bereich der Teilchengröße der Kerne aufweisen und oder sogar unterhalb davon, stört dies die Gitterordnungen nach vorliegenden Untersuchungen nicht. Es hat sich auch gezeigt, dass die Form der eingelagerten Kontrastmaterialpartikel keinen oder nur geringen Einfluss auf den optischen Effekt hat. Es können erfindungsgemäß sowohl kugelförmige als auch plättchenförmige und nadelförmige Kontrastmaterialien eingelagert werden. Von Bedeutung scheint lediglich die absolute Teilchengröße im Verhältnis zur Teilchengröße der Kerne zu sein. Daher ist es erfindungsgemäß bevorzugt, wenn die Teilchengröße des mindestens einen Kontrastmaterials mindestens doppelt so groß ist wie die Teilchengröße des Kernmaterials, wobei die Teilchengröße des mindestens einen Kontrastmaterials vorzugsweise mindestens viermal so groß ist wie die Teilchengröße des Kernmaterials, da dann die beobachtbaren Wechselwirkungen noch geringer sind.

Eine sinnvolle Obergrenze der Teilchengröße der Kontrastmaterialien ergibt sich aus der Grenze, bei der die einzelnen Partikel selbst sichtbar werden oder aufgrund ihrer Teilchengröße die mechanischen Eigenschaften des Beugungsfarbmittels beeinträchtigen. Die Bestimmung dieser Obergrenze bereitet dem Fachmann keinerlei Schwierigkeiten.

Von Bedeutung für den erfindungsgemäß erwünschten Effekt ist außerdem die Menge an Kontrastmaterial, die eingesetzt wird. Es hat sich gezeigt, dass Effekte üblicherweise beobachtet werden, wenn mindestens 0,05 Gew.-% Kontrastmaterial, bezogen auf das Gewicht des Beugungsfarbmittel als eingesetzt werden. Besonders bevorzugt ist es, wenn der Beugungsfarbmittel mindestens 0,2 Gew.-% und insbesondere bevorzugt mindestens 1 Gew.-% Kontrastmaterial enthält, da diese erhöhten Gehalte an Kontrastmaterial erfindungsgemäß in der Regel auch zu intensiveren Effekten führen.

Umgekehrt beeinträchtigen größere Mengen an Kontrastmaterial unter Umständen die Verarbeitungseigenschaften der Kern/Mantel-Partikel und erschweren so die Herstellung erfindungsgemäßer Beugungsfarbmittel. Darüber hinaus wird erwartet, dass oberhalb eines gewissen Anteils von Kontrastmaterial, der vom jeweiligen Material abhängt, die Ausbildung des Gitters aus Kern-Partikeln gestört wird und sich vielmehr orientierte Kontrastmaterialschichten bilden. Daher ist es erfindungsgemäß bevorzugt, wenn der Beugungsfarbmittel maximal 20 Gew.-% Kontrastmaterial, bezogen auf das Gewicht des Beugungsfarbmittels, enthält, wobei es besonders bevorzugt ist, wenn der Beugungsfarbmittel maximal 12 Gew.-% und insbesondere bevorzugt maximal 5 Gew.-% Kontrastmaterial enthält.

In einer besonderen Ausführungsform der vorliegenden Erfindung kann es jedoch auch bevorzugt sein, wenn die Beugungsfarbmittel möglichst große Mengen an Kontrastmaterial enthalten. Das ist insbesondere dann der Fall, wenn das Kontrastmaterial gleichzeitig die mechanische Festigkeit des Beugungsfarbmittels erhöhen soll.

Vorzugsweise handelt es sich bei dem erfindungsgemäßen Beugungsfarbmittel um plättchenförmige Partikel, deren Dicke vorzugsweise im Bereich von 0,5 bis 20 $\mu$m, insbesondere bevorzugt im Bereich 1 bis 10 $\mu$m liegt, wobei in dem Plättchen in jeder Raumrichtung mindestens 5, vorzugsweise mindestens 8 und insbesondere bevorzugt mindestens 10 Kern-Lagen aufeinander folgen.

Die erfindungsgemäßen Beugungsfarbmittel können dabei durch Mahlen oder Brechen aus Filmen von Kern-Mantel-Partikeln, deren Herstellung im Prinzip in DE-A-10145450 beschrieben ist erhalten werden. Folglich ist ein Verfahren zur Herstellung von Beugungsfarbmitteln geeignet, bei dem aus Kern-Mantel-Partikeln, deren Kern im wesentlichen fest ist und eine im wesentlichen monodisperse Größenverteilung aufweist, wobei ein Unterschied zwischen den Brechungsindices des Kernmaterials und des Mantelmaterials besteht, ein Film hergestellt wird, dessen Matrix spröde ist, und der Film anschließend zu Partikeln zerkleinert, vorzugsweise gemahlen wird.

In einem bevorzugten Verfahren werden dabei Kern-Mantel-Partikel eingesetzt, deren Mantel aus Polymeren die eine Glasübergangstemperatur $T_G$ von oberhalb 25°C, vorzugsweise oberhalb von 50°C und insbesondere bevorzugt oberhalb von 70°C aufweisen. Entsprechende Polymere sind dem Fachmann wohl bekannt und finden sich beispielsweise in Brandrup, J. (Ed.).: Polymer Handbook. Chichester Wiley 1966. Bevorzugte Mantelmaterialien sind dabei homo- oder copolymeres Poly(cyclohexylmethacrylat), Polystyrol sowie substituierte Polystyrolderivate, wie z. B. Poly(iodstyrol) und Poly(bromstyrol), Polyacrylate und Polymethacrylate mit einer Tg oberhalb der Gebrauchstemperatur, Polyvinylchlorid mit hoher Tg und andere vinylische Polymere, die sich durch Umwandlung aus Polyvinylacetat ergeben, Polyacrylnitril und Styrol-Acrylnitril-Copolymere.

In einem anderen bevorzugten Verfahren werden Kern-Mantel-Partikel eingesetzt, deren Mantel aus Polymeren besteht, die eine vernetzbare Funktionalität aufweisen. Bei dieser Verfahrensvariante erfolgt vorzugsweise vor der Herstellung des Filmes eine Additivierung der Kern-Mantel-Partikel mit nachvernetzenden Additiven und nach der Herstellung des Filmes die eigentliche Vernetzung. Dabei können als Nachvernetzer bzw. vernetzbare Funktionalität insbesondere die oben beschriebenen Verbindungen bzw. Monomere eingesetzt werden. Die Vernetzung kann dabei nach den beschriebenen Methoden gestartet werden, wobei die thermische Aktivierung zur Nachvernetzung insbesondere bevorzugt ist.

Bei der Herstellung der Filme wird zuerst eine Mischung aus Kern-Mantel-Partikeln und ggf. weiteren Additiven hergestellt. Vorzugsweise wird dann die Mischung bei einer Temperatur, bei der der Mantel fließfähig ist einer mechanischen Kraft ausgesetzt.

In einer bevorzugten Variante der Herstellung erfindungsgemäßer Filme liegt die Temperatur bei der die Mischung der mechanischen Kraft ausgesetzt wird mindestens 40°C, vorzugsweise mindestens 60°C oberhalb des Glaspunktes des Mantels der Kern-Mantel-Partikel. Es hat sich empirisch gezeigt, dass die Fließfähigkeit des Mantels in diesem Temperaturbereich den Anforderungen für eine wirtschaftliche Herstellung der Filme in besonderem Maße entspricht.

In einer ebenfalls bevorzugten Verfahrensvariante, die zu erfindungsgemäßen Filmen führt, werden die fließfähigen Mischungen unter Einwirkung der mechanischen Kraft auf eine Temperatur abgekühlt, bei der der Mantel nicht mehr fließfähig ist.

Bei der mechanischen Krafteinwirkung kann es sich erfindungsgemäß um eine solche Krafteinwirkung handeln, die bei üblichen Verarbeitungsschritten von Polymeren erfolgt. In bevorzugten Varianten der vorliegenden Erfindung erfolgt die mechanische Krafteinwirkung entweder:

- durch uniaxiales Pressen oder
- während eines Transferpressvorganges,
- während einer (Co-) Extrusion oder
- während eines Kalandriervorganges oder
- während eines Blasvorganges.

Erfolgt die Krafteinwirkung durch uniaxiales Pressen, so handelt es sich bei den erfindungsgemäßen Filmen vorzugsweise um Filme. Erfindungsgemäße Filme können dabei vorzugsweise auch durch Kalandrieren, Folienblasen oder Flachfolienextrusion hergestellt werden. Die verschiedenen Möglichkeiten der Verarbeitung von Polymeren unter Einwirkung mechanischer Kräfte sind dem Fachmann wohl bekannt und können beispielsweise dem Standardlehrbuch Adolf Franck, "Kunststoff-Kompendium"; Vogel-Verlag; 1996 entnommen werden.

Dabei können die erfindungsgemäßen Filme, wenn es technisch vorteilhaft ist, Hilfs- und Zusatzstoffe enthalten. Sie können der optimalen Einstellung der für die Anwendung und Verarbeitung gewünschten bzw. erforderlichen anwendungstechnischen Daten, bzw. Eigenschaften dienen. Beispiele für derartige Hilfs- und/oder Zusatzstoffe sind Weichmacher, Filmbildungshilfsmittel, Verlaufmittel, Füllmittel, Schmelzhilfsmittel, Haftmittel, Trennmittel, Auftragshilfsmittel, Mittel zur Viskositätsmodifizierung, z. B. Verdicker.

Besonders empfehlenswert sind Zusätze von Filmbildungshilfsmitteln und Filmmodifizierungsmitteln auf der Basis von Verbindungen der allgemeinen Formel $HO-C_nH_{2n}-O-(C_nH_{2n}-O)_mH$, worin n eine Zahl von 2 bis 4, vorzugsweise 2 oder 3, und m eine Zahl von 0 bis 500 ist. Die Zahl n kann innerhalb der Kette variieren und die verschiedenen Kettenglieder können in statistischer oder in blockweiser Verteilung eingebaut sein. Beispiele für derartige Hilfsmittel sind Ethylenglycol, Propylenglycol, Di-, Tri- und Tetraethylenglycol, Di-, Tri- und Tetrapropylenglycol, Polyethylenoxide, Polypropylenoxid und Ethylenoxid/Propylenoxid-Mischpolymere mit Molgewichten bis ca. 15000 und statistischer oder blockartigen Verteilung der Ethylenoxid und Propylenoxid-Baugruppen.

Gegebenenfalls sind auch organische oder anorganische Lösungs-, Dispergier- oder Verdünnungsmittel, die beispielsweise die offene Zeit der Formulierung, d. h. die für ihren Auftrag auf Substrate zur Verfügung stehende Zeit, verlängern, Wachse oder Schmelzkleber als Additive möglich.

Gewünschtenfalls können den Filmen auch Stabilisatoren gegen UV-Strahlung und Wettereinflüsse zugesetzt werden. Hierzu eignen sich z. B. Derivate des 2,4-Dihydroxybenzophenons, Derivate des 2-Cyan-3,3'-dephenylacrylats, Derivate des 2,2',4,4'-Tetrahydroxybenzophenons, Derivate des o-Hydroxyphenyl-benztriazols, Salicylsäureester, o-Hydroxyphenyl-s-triazine oder sterisch gehinderte Amine. Auch diese Stoffe können einzeln oder als Gemische eingesetzt werden.

Die Gesamtmenge der Hilfs- und/oder Zusatzstoffe beträgt bis zu 40 Gew.-%, vorzugsweise bis zu 20 Gew.-%, insbesondere bevorzugt bis zu 5 Gew.-% des Gewichts der Filme. Dementsprechend bestehen die Filme zu mindestens 60 Gew.-%, vorzugsweise zu mindestens 80 Gew.-% und insbesondere bevorzugt zu mindestens 95 Gew.-% aus Kern-Mantel-Partikeln.

Diese Filme können als erfindungsgemäße Beugungsfarbmittel eingesetzt werden. Sollen partikuläre Beugungsfarbmittel hergestellt werden, so gelingt dies indem die Filme durch Schneiden, Brechen und/oder Mahlen zu Pigmenten geeigneter Größe zerkleinert werden. Dieser Vorgang kann beispielsweise in einem kontinuierlichen Bandverfahren erfolgen. Es kann ein Walzwerk oder ein Mahlwerk eingesetzt werden. Sind besonders gleichmäßige Teilchengrößen bei den Beugungsfarbmitteln erwünscht, so können sich an den Zerkleinerungsschritt noch ein oder mehrere Siebschritte anschließen.

Die inverse Struktur zur Opalstruktur entsteht gedanklich dadurch, dass in einem massiven Material regelmäßige sphärische Hohlvolumina in einer dichtesten Packung angeordnet werden. Ein Vorteil von derartigen inversen Strukturen gegenüber den normalen Strukturen ist das Entstehen von photonischen Bänderlücken bei bereits viel geringeren Dielektrizitätskonstantenkontrasten (K. Busch et al. Phys. Rev. Letters E, 198, 50, 3896).

Beugungsfarbmittel die Kavitäten aufweisen müssen folglich eine feste Matrix besitzen. In einer Erfindungsvariante besteht die Matrix im wesentlichen aus einem organischen Polymer, das vorzugsweise vernetzt ist. In einer anderen Erfindungsvariante besteht die Matrix um die Kavitäten im wesentlichen aus einem anorganischen Material, vorzugsweise einem Metall oder Halbmetall oder einem Metallchalcogenid oder Metallpnictid bestehen, wobei insbesondere Silciumdioxid, Aluminiumoxid, Zirkonoxid, Eisenoxide, Titandioxid, Cerdioxid, Galliumnitrid, Bor- und Aluminiumnitrid sowie Silicium- und Phosphornitrid oder Mischungen davon bevorzugt sind.

Dreidimensionale inverse Strukturen d. h. erfindungsgemäß einzusetzende Beugungsfarbmittel mit regelmäßigen Anordnungen von Kavitäten können beispielsweise durch eine Templatsynthese hergestellt werden:

- Als Struktur gebende Template werden monodisperse Kugeln in einer dichtesten Kugelpackung angeordnet.
- Die Hohlvolumina zwischen den Kugeln werden durch Ausnutzung von Kapillareffekten mit einem gasförmigen oder flüssigen Precursor oder einer Lösung eines Precursors befüllt.
- Der Precursor wird (thermisch) in das gewünschte Material umgesetzt.
- Die Template werden entfernt, wobei die inverse Struktur zurückbleibt.

In der Literatur sind viele solcher Verfahren bekannt. Beispielsweise können $SiO_2$-Kugeln in eine dichteste Packung arrangiert werden, die Hohlvolumina mit Tetraethylorthotitanat enthaltenden Lösungen befüllt werden. Nach mehreren Temperschritten werden in einem Ätzprozess mit HF die Kugeln entfernt, wobei die inverse Struktur aus Titandioxid zurückbleibt (V. Colvin et al. Adv. Mater. 2001, 13, 180).

De La Rue et al. (De La Rue et al. Synth. Metals, 2001, 116, 469) beschreiben die Herstellung von inversen, aus $TiO_2$ bestehenden Opalen nach folgender Methodik: Eine Dispersion von 400 nm großen Polystyrolkugeln wird auf einem Filterpapier unter einer IR-Lampe getrocknet. Der Filterkuchen wird mit Ethanol abgesaugt, in eine Glovebox überführt und mittels einer Wasserstrahlpumpe mit Tetraethylorthotitanat infiltriert. Das Filterpapier von dem Latex-Ethoxid-Komposit vorsichtig entfernt und der Komposit in einen Rohrofen überführt. In dem Rohrofen findet bei 575 °C die 8 h dauernde Calzinierung in einem Luftstrom statt, wodurch aus dem Ethoxid Titandioxid gebildet wird und die Latexpartikel herausgebrannt werden. Es bleibt eine inverse Opalstruktur aus $TiO_2$ zurück.

Martinelli et al. (M. Martinelli et al. Optical Mater. 2001, 17, 11) beschreiben die Herstellung von invesen $TiO_2$-Opalen mittels Verwendung von 780 nm und 3190 nm großen Polystyrolkugeln. Eine regelmäßige Anordnung in einer dichtesten Kugelpackung wird durch 24 -48-stündiges Zentrifugieren der wässrigen Kugeldispersion bei 700 - 1000 U/min und nachfolgendes Dekantieren, gefolgt von Lufttrocknung erreicht. Die regelmäßig angeordneten Kugeln werden auf einem Filter auf einem Büchnertrichter mit Ethanol angefeuchtet und dann tropfenweise mit einer ethanolischen Lösung von Tetraethylorthotitanat versehen. Nach einsickern der Titanatlösung wird die Probe in einem Vakuumexsikkator über 4 - 12 Stunden getrocknet. Diese Befüllungsprozedur wird 4 bis 5-mal wiederholt. Die Polystyrolkugeln werden anschließend bei 600 °C - 800 °C über 8 - 10 Stunden herausgebrannt.

Stein et al. (A. Stein et al. Science, 1998, 281, 538) beschreiben die Synthese von inversen $TiO_2$-Opalen ausgehend von Polystyrolkugeln eines Durchmessers von 470 nm als Template. Diese werden in einem 28-stündigem Prozess hergestellt, einer Zentrifugierung unterzogen und Luft getrocknet. Danach werden die LaticesTemplate auf ein Filterpapier aufgebracht. In das Latextemplate wird über einen Büchnertrichter, der an eine Vakuumpumpe angeschlossen ist, Ethanol eingesogen. Danach erfolgt tropfenweise Zugabe von Tetraethylorthotitanat unter Absaugen. Nach Trocknen im Vakuum Exsikkator über 24 h werden die Latices bei 575 °C über 12 h im Luftstrom herausgebrannt.

Vos et al. (W. L. Vos et al. Science, 1998, 281, 802) stellen inverse $TiO_{2}$-Opale her, indem sie Polystyrolkugeln mit Durchmessern von 180 - 1460 nm als Template verwenden. Zur Einstellung der dichtesten Kugelpackung der Kugeln wird eine Sedimentationstechnik verwendet, die mit Zentrifugieren über einen Zeitraum von bis zu 48 h unterstützt wird. Nach langsamen Evakuieren zur Trocknung der Templatstruktur wird diese in einer Glovebox mit einer ethanolischen Lösung von Tetra-n-propoxyorthotitanat versetzt. Nach ca. 1 h wird das infiltrierte Material an die Luft gebracht, um den Precursor zu $TiO_2$ reagieren zu lassen. Diese Prozedur wird achtmal wiederholt, um eine vollständige Füllung mit $TiO_2$ zu gewährleisten. Danach wird das Material bei 450 °C calziniert.

Kern-Mantel-Partikeln, deren Mantel eine Matrix bildet und deren Kern im wesentlichen fest ist und eine im

wesentlichen monodisperse Größenverteilung aufweist, sind in der Deutschen Patentanmeldung DE-A-10145450 beschrieben. Die Verwendung solcher Kern-Mantel-Partikel, deren Mantel eine Matrix bildet und deren Kern im wesentlichen fest ist und eine im wesentlichen monodisperse Größenverteilung aufweist als Template zur Herstellung inverser Opalstrukturen und ein Verfahren zur Herstellung inverser opalartiger Strukturen unter Einsatz solcher Kern-Mantel-Partikel ist in der Internationalen Patentanmeldung WO 2004/031102 beschrieben. Die beschriebenen Formkörper mit homogenen, regelmäßig angeordneten Kavitäten (d.h. inverser Opalstruktur) besitzen vorzugsweise Wände aus Metalloxiden oder aus Elastomeren. Folglich sind die beschriebenen Formkörper entweder hart und spröde oder zeigen elastomeren Charakter.

Aus der älteren Deutschen Patentanmeldung mit dem Anmeldeaktenzeichen DE 10341198.4 ist Verwendung von Kern-Mantel-Partikeln deren Mantel thermoplastische Eigenschaften aufweist, zur Herstellung von Formkörpern mit homogenen, regelmäßig angeordneten Kavitäten deren mechanische Eigenschaften besonders vorteilhaft sind, beschrieben.

Kern-Mantel-Partikel, deren Mantel eine Matrix bildet und deren Kern im wesentlichen fest ist und eine im wesentlichen monodisperse Größenverteilung aufweist und deren Mantel mit dem Kern über eine Zwischenschicht verbunden ist und deren Mantel thermoplastische Eigenschaften aufweist, werden dabei zur Herstellung von Formkörpern mit homogenen, regelmäßig angeordneten Kavitäten verwendet. Das entsprechende Verfahren zur Herstellung von Formkörpern mit homogenen, regelmäßig angeordneten Kavitäten, ist dadurch gekennzeichnet, dass Kern-Mantel-Partikel, deren Mantel eine Matrix bildet und deren Kern im wesentlichen fest ist und eine im wesentlichen monodisperse Größenverteilung aufweist und mit dem Kern über eine Zwischenschicht verbunden ist und deren Mantel thermoplastische Eigenschaften aufweist, unter Anwendung einer mechanischen Kraft und erhöhter Temperatur zu Formkörpern, vorzugsweise Filmen verarbeitet werden und anschließend die Kerne entfernt werden.

Es resultieren Formkörper mit homogenen, regelmäßig angeordneten Kavitäten, die dadurch gekennzeichnet sind, dass die regelmäßig angeordneten Kavitäten in eine Matrix mit thermoplastischen oder duroplastischen Eigenschaften eingebettet sind.

Dabei ist es aufgrund der leichteren Verarbeitbarkeit in einer Variante insbesondere bevorzugt, wenn die regelmäßig angeordneten Kavitäten in eine Matrix mit thermoplastischen Eigenschaften eingebettet sind, wobei die Matrix vorzugsweise aus Poly(styrol), thermoplastischen Poly(acrylat)-derivaten, vorzugsweise Poly(methylmethacrylat) oder Poly(cyclohexylmethacrylat), bzw. thermoplastischen Copolymeren dieser Polymere mit anderen Acrylaten, wie vorzugsweise Styrol-Acrylnitril-Copolymeren, Styrol-Ethylacrylat-Copolymeren oder Methylmethacrylat-Ethylacrylat) -Copolymeren aufgebaut ist.

In einer anderen bevorzugten Ausführungsform besteht in den Kern-Mantel-Partikeln der Kern im wesentlichen aus einem mit UV-Strahlung abbaubaren Material, vorzugsweise einem UV-abbaubaren organischen Polymeren und insbesondere bevorzugt aus Poly(tert-butylmethacrylat), Poly(methylmethacrylat), Poly(n-butylmethacrylat) oder Copolymeren, die eines dieser Polymere enthalten.

Der Mantel der Kern-Mantel-Partikel besteht dabei vorzugsweise aus dem Material das später die Matrix um die Kavitäten bildet.

Die Entfernung der Kerne kann auf verschiedenen Wegen erfolgen. Wenn die Kerne aus geeigneten anorganischen Materialien bestehen, können diese durch Ätzen entfernt werden. Vorzugsweise können zum Beispiel Siliciumdioxid-Kerne mit HF, insbesondere verdünnter HF-Lösung entfernt werden. Bei diesem Vorgehen kann es wiederum bevorzugt sein, wenn vor oder nach der Entfernung der Kerne eine Vernetzung des Mantels erfolgt. In diesem Fall erhält der Mantel und damit die Matrix des Formkörpers duroplastische Eigenschaften. Wenn die Kerne in den Kern-Mantel-Partikeln aus einem mit UV-Strahlung abbaubaren Material, vorzugsweise einem UV-abbaubaren organischen Polymeren aufgebaut sind, erfolgt die Entfernung der Kerne durch UV-Bestrahlung. Auch bei diesem Vorgehen kann es wiederum bevorzugt sein, wenn vor oder nach der Entfernung der Kerne eine Vernetzung des Mantels erfolgt.

Die Verwendung von Kern-Mantel-Partikeln, deren Mantel eine Matrix bildet und deren Kern im wesentlichen aus einem abbaubaren Polymeren besteht und eine im wesentlichen monodisperse Größenverteilung aufweist und deren Mantel zu einer Carbon-Matrix pyrolysiert werden kann, zur Herstellung von Formkörpern mit regelmäßig angeordneten Kavitäten, ein Verfahren zur Herstellung von Formkörpern mit regelmäßig angeordneten Kavitäten und die entsprechenden Formkörper werden in der älteren Deutschen Patentanmeldung DE 10357681.9 beschrieben.

Unter einer Carbon-Matrix werden dabei Materialien verstanden, die denen von Carbon-Fasern weitgehend entsprechen. In einem Extremfall handelt es sich bei der erfindungsgemäßen Carbon-Matrix um elementaren Kohlenstoff, vorzugsweise amorpher oder teilkristalliner Form, wobei die kristallinen Anteile in der Graphit-Modifikation oder Graphit ähnlichen Modifikationen, wie Fullerenen, Carbon-Nanotubes und ähnlichen Graphitartigen Strukturen vorliegen. In einer anderen extremen Ausprägung der vorliegenden Erfindung handelt es sich bei der Carbon-Matrix um Leiterpolymere, wie beispielsweise Polyimide, die sich bei der thermischen Kondensation von Polyacrylnitril bilden. Im Regelfall handelt es sich bei der Carbon-Matrix jedoch um ein Material, dessen chemischer Aufbau zwischen diesen beiden extremen liegt. Es wird vermutet, dass ähnlich der Situation bei Rußen in den Materialen wechselnde Anteile polycyclischer aromatischer Kohlenwasserstoffe vorliegen können, die mit imidischen Funktionen versehen sind.

Um die Bildung der Carbon-Matrix zu erleichtern ist es erfindungsgemäß insbesondere bevorzugt, wenn in den Kern-Mantel-Partikeln der Mantel aus im wesentlichen unvernetzten organischen Polymeren besteht, die über eine zumindest teilweise vernetzte Zwischenschicht auf den Kern aufgepfropft sind, wobei der Mantel vorzugsweise im wesentlichen aus Polyacrylnitril (PAN) bzw. Polymethacrylnitril oder Copolymeren, die Polyacrylnitril bzw. Polymethacrylnitril enthalten, wie Polystyrolacrylnitril (PSAN), gebildet wird. Dabei zersetzt PAN sich bereits bei Temperaturen von 250-280°C zu einer geeigneten Carbon-Matrix.

Das Verfahren zur Herstellung von Formkörpern mit regelmäßig angeordneten Kavitäten gemäß DE 10357681.9, ist dadurch gekennzeichnet, dass Kern-Mantel-Partikel, deren Mantel eine Matrix bildet und deren Kern im wesentlichen aus einem abbaubaren Polymeren besteht und eine im wesentlichen monodisperse Größenverteilung aufweist und deren Mantel zu einer Carbon-Matrix pyrolysiert werden kann, unter Anwendung einer mechanischen Kraft und erhöhter Temperatur zu Formkörpern, vorzugsweise Filmen verarbeitet werden und anschließend die Kerne durch Abbau entfernt werden und der Mantel bei erhöhter Temperatur zu einer Carbon-Matrix zersetzt wird.

Dabei ist es insbesondere bevorzugt, wenn in den Kern-Mantel-Partikeln der abbaubare Kern thermisch abbaubar ist und aus Polymeren besteht, die entweder thermisch depolymerisierbar sind, d.h. unter Temperatureinwirkung in ihre Monomere zerfallen oder der Kern aus Polymeren besteht, die beim Abbau in niedermolekulare Bestandteile zerfallen, die von den Monomeren verschieden sind. Wichtig ist dabei, dass der Abbau der Kernpolymere bei einer Temperatur erfolgt die gleich oder niedriger ist als die Temperatur bei der sich die Carbon-Matrix bildet. Geeignete Polymere finden sich beispielsweise in der Tabelle "Thermal Degradation of Polymers" in Brandrup, J. (Ed.).: Polymer Handbook.

Chichester Wiley 1966, S. V-6 - V-10, wobei alle Polymere geeignet sind, die flüchtige Abbauprodukte liefern. Der Inhalt dieser Tabelle gehört ausdrücklich zur Offenbarung der vorliegenden Anmeldung.

Geeignete thermisch abbaubare Polymere sind insbesondere

- Poly(styrol) und Derivate, wie Poly(α-methylstyrol) bzw. Poly(styrol)-derivate, die am aromatischen Ring Substituenten tragen, wie insbesondere teil- oder perfluorierte Derivate,
- Poly(acrylat)- und Poly(methacrylat)-derivate sowie deren Ester, insbesondere bevorzugt Poly(methylmethacrylat) oder Poly(cyclohexylmethacrylat), bzw. Copolymeren dieser Polymere mit anderen abbaubaren Polymeren, wie vorzugsweise Styrol-Ethylacrylat-Copolymeren oder Methylmethacrylat-Ethylacrylat-Copolymeren,
- Polybutadien und Copolymere mit anderen der hier genannten Monomere,
- Cellulose und Derivate wie oxidierte Cellulose und Cellulosetriacetat,
- Polyketone, wie z.B. Poly(methylisopropenylketon) oder Poly(methylvinylketon),
- Polyolefine, wie z.B. Polyethylen und Polypropylen, Polyisisopren, Polyolefinoxide, wie zu.B. Polyethylenoxid oder Polypropylenoxid, Polyethylenterephthalat, Polyformaldehyd, Polyamide, wie Nylon 6 und Nylon 66, Polyperfluoroglucarodiamidin, Plolperfluorpolyolefine, wie Plolyperfluorpropylen, Polyperfluorohepten,
- Polyvinylacetat, Polyvinylchlorid, Polyvinylalkohol, Polyvinylcyclohexanon, Polyvinylbutyrat, Polyvinylfluorid

Insbesondere bevorzugt ist dabei der Einsatz von Poly(styrol) und Derivaten, wie Poly(α-methylstyrol) bzw. Poly(styrol)-derivate, die am aromatischen Ring Substituenten tragen, wie insbesondere teil- oder perfluorierte Derivaten, Poly(acrylat)- und Poly(methacrylat)-derivaten sowie deren Estern, insbesondere bevorzugt Poly(methylmethacrylat) oder Poly(cyclohexylmethacrylat), bzw. Copolymeren dieser Polymere mit anderen abbaubaren Polymeren, wie vorzugsweise Styrol-Ethylacrylat-Copolymeren oder Methylmethacrylat-Ethylacrylat-Copolymeren, und Polyolefinen, Polyolefinoxiden, Polyethylenterephthalat, Polyformaldehyd, Polyamiden, Polyvinylacetat, Polyvinylchlorid oder Polyvinylalkohol.

Soll der Kern der Kern-Mantel-Partikel durch UV-Strahlung abgebaut werden, so wird der Kern im wesentlichen von einem mit UV-Strahlung abbaubaren Material, vorzugsweise einem UV-abbaubaren organischen Polymeren und insbesondere bevorzugt aus Poly(tert-butylmethacrylat), Poly(methylmethacrylat), Poly(n-butylmethacrylat) oder Copolymeren, die eines dieser Polymere enthalten, aufgebaut.

Die Verwendung der bei der Herstellung gemäß der Lehre der WO 2004/031102, DE 10357681.9 bzw. DE 10341198.4 resultierenden Formkörper in der Kosmetik ist jetzt einer der Gegenstände der vorliegenden Anmeldung. Hinsichtlich der Beschreibung der Formkörper und der Herstellverfahren für Formkörper wird auf die Patentanmeldungen WO 2004/031102 und DE 10341198.4 verwiesen, deren entsprechende Offenbarung ausdrücklich auch zum Inhalt der vorliegenden Anmeldung gehört.

Insbesondere bevorzugt ist es erfindungsgemäß, wenn der mittlere Durchmesser der Kavitäten in den Formkörpern im Bereich von etwa 50 - 500 nm, bevorzugt im Bereich von 100 - 500 nm und ganz besonders bevorzugt im Bereich von 200 bis 280 nm liegt.

Die Formkörper fallen bei den entsprechenden Verfahren entweder direkt in Pulverform an oder können durch Mahlen zerkleinert werden. Die resultierenden Partikel finden dann im erfindungsgemäßen Sinne als Beugungsfarbmittel zur dekorativen Anwendung in Kosmetischen Zubereitungen oder auf der Haut Anwendung.

Die Konzentration des Beugungsfarbmittels in dem zu pigmentierenden Anwendungssystem d.h. der Dispersion liegt in der Regel zwischen 0,1 und 70 Gew.-%, vorzugsweise zwischen 0,1 und 50 Gew.-% und insbesondere zwischen 1,0 und 20 Gew.-%, bezogen auf den Gesamtfestkörpergehalt des Systems.

Ein weiterer Gegenstand der vorliegenden Erfindung sind Zubereitungen zur topischen Anwendung enthaltend mindestens ein Beugungsfarbmittel und mindestens einen zur topischen Anwendung geeigneten Träger. Dabei sind Zubereitungen bevorzugt, in denen die Gesamtmenge des oder der Beugungsfarbmittel aus dem Bereich von 0,1 Gew.-% bis 30 Gew.-%, bezogen auf das Gesamtgewicht der Zubereitung, gewählt wird.

Ferner können die erfindungsgemäßen Zubereitungen auch weitere Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| Chemische oder sonstige Bezeichnung | CIN | Farbe |
|---|---|---|
| Pigment Green | 10006 | grün |
| Acid Green 1 | 10020 | Grün |
| 2,4-Dinitrohydroxynaphthalin-7-sulfonsäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'sulfonsäurediethylamid-1'-phenylazo)-3-hydroxy-5"-chloro-2", 4"-dimethoxy2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydroxynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydroxy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |

Tabelle fortgesetzt

| Chemische oder sonstige Bezeichnung | CIN | Farbe |
|---|---|---|
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2-phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |
| Pigment Yellow 16 | 20040 | Gelb |
| 2,6-(4'-Sulfo-2",4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1 "-phenylazo)-7'-sulfo-1'-naphthylazo]-1-hydroxy-7-aminonaphthalin-3,6-disulfosäure | 27755 | Schwarz |
| 4-[4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd ($C_{30}$) | 40820 | Orange |
| trans-Apo-8'-Carotinsäure ($C_{30}$)-ethylester | 40850 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N- diethyl)-amino-2-methyl-N-ethylIN-m-sulfobenzylfuchsonimmonium | 42735 | Blau |
| 4'-(N-Dimethyl)-amino-4"-(N-phenyl)-aminonaphtho-N-dimethylfuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl) -xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |

Tabelle fortgesetzt

| Chemische oder sonstige Bezeichnung | CIN | Farbe |
|---|---|---|
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |
| Acid Violet 50 | 50325 | Violett |
| Acid Black 2 | 50420 | Schwarz |
| Pigment Violet 23 | 51319 | Violett |
| 1,2-Dioxyanthrachinon, Calcium-Sluminiumkomplex | 58000 | Rot |
| 3-Oxypyren-5,8,10-sulfosäure | 59040 | Grün |
| 1-Hydroxy-4-N-phenyl-aminoanthrachinon | 60724 | Violett |
| 1-Hydroxy-4-(4'-methylphenylamino)-anthrachinon | 60725 | Violett |
| Acid Violet 23 | 60730 | Violett |
| 1,4-Di(4'-methyl-phenylamino)-anthrachinon | 61565 | Grün |
| 1,4-Bis-(o-sulfo-p-toluidino)-anthrachinon | 61570 | Grün |
| Acid Blue 80 | 61585 | Blau |
| Acid Blue 62 | 62045 | Blau |
| N,N'-Dihydro-1,2,1',2'-anthrachinonazin | 69800 | Blau |
| Vat Blue 6; Pigment Blue 64 | 69825 | Blau |
| Vat Orange 7 | 71105 | Orange |
| Indigo | 73000 | Blau |
| Indigo-disulfosäure | 73015 | Blau |
| 4,4'-Dimethyl-6,6'-dichlorthioindigo | 73360 | Rot |
| 5,5'Dichlor-7,7'-dimethylthioindigo | 73385 | violett |
| Quinacridone Violet 19 | 73900 | violett |
| Pigment Red 122 | 73915 | Rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6, 19; Natural Red 1 | 75100 | gelb |
| Bixin, Nor-Bixin | 75120 | orange |
| Lycopin | 75125 | gelb |
| trans-alpha-, bet- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na, Al, Ca) der Karminsäure | 75470 | Rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | Rot |
| Bariumsulfat | 77120 | weiß |

Tabelle fortgesetzt

| Chemische oder sonstige Bezeichnung | CIN | Farbe |
|---|---|---|
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268 :1 | schwarz |
| Chromoxid | 77288 | grün |
| Chromoxid, wasserhaltig | 77278 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyahoferrat | 77510 | blau |
| Pigment White 18 | 77713 | weiß |
| Mangananimoniumdiphosphat | 77742 | violett |
| Manganphosphat; $Mn_3(PO_4)_2 \cdot 7\ H_2O$ | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalzem, Anthocyane | | rot |
| Aluminium-, Zink-, Magnesium- und Calciumstearat | | weiß |
| Bromthymolblau | | blau |

Es kann ferner günstig sein, als Farbstoff eine oder mehrerer Substanzen aus der folgenden Gruppe zu wählen: 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aiuminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, β-Carotin oder Cochenille.

Vorteilhaft im Sinne der vorliegenden Erfindung sind ferner Gelcrèmes mit einem Gehalt an Perlglanzpigmenten. Bevorzugt sind insbesondere die im folgenden aufgelisteten Arten von Perlglanzpigmenten:

1. Natürliche Perlglanzpigmente, wie z. B.

    1. "Fischsilber" (Guanin/Hypoxanthin-Mischkristalle aus Fischschuppen) und

2. "Perlmutt" (vermahlene Muschelschalen)

2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung/Schichtdicke | Farbe |
|---|---|---|
| **Silberweiße Perlglanzpigmente** | $TiO_2$: 40-60 nm | silber |
| **Interferenzpigmente** | $TiO_2$: 60-80 nm | gelb |
| | $TiO_2$: 80-100 nm | rot |
| | $TiO_2$: 100-140 nm | blau |
| | $TiO_2$: 120-160 nm | grün |
| **Farbglanzpigmente** | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| **Kombinationspigmente** | $TiO_2$ / $Fe_2O_3$ | Goldtöne |
| | $TiO_2$ / $Cr_2O_3$ | grün |
| | $TiO_2$ / Berliner Blau | tiefblau |

Besonders bevorzugt sind z. B. die von der Firma Merck unter den Handelsnamen Timiron, Colorona, Dichrona, Xirona und Ronastar erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit $TiO_2$ und $Fe_2O_3$ beschichtete $SiO_2$-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Perlglanzpigmente, welche unter der Verwendung von $SiO_2$ hergestellt werden. Solche Pigmente , die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihre Partikelgröße von 40-80 $\mu$m zusätzlich zu der Farbe einen Glitzereffekt auf.

Besonders vorteilhaft sind ferner auch Effektpigmente, welche unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.% bis 30 Gew.%, vorzugsweise von 0,5 bis 15 Gew.%, insbesondere von 1,0 bis 10 Gew.% gewählt, jeweils bezogen auf das Gesamtgewicht der Zubereitungen.

Erfindungsgemäße Zubereitungen bzw. erfindungsgemäß zu verwendende Zubereitungen können weiter mindestens ein Repellent enthalten, wobei das Repellent vorzugsweise ausgewählt aus N,N-Diethyl-3-methylbenzamid,

3-(Acetyl-butyl-amino)-propionsäure Ethylester, Dimethylphthalat, Butopyronoxyl, 2,3,4,5-bis-(2-Butylen)-tetrahydro-2-furaldehyd, N,N-Caprylsäurediethylamid, N,N-Diethylbenzamid, o-Chlor-N,N-diethylbenzamid, Dimethylcarbat, Di-n-propylisocinchomeronat, 2-Ethylhexan-1,3-diol, N-Octyl-bi-cyclohepetendiecarboximid, Piperonylbutoxid, 1-(2-Methylpropyloxycarbonyl)-2-(hydroxyethyl)-piperidin, Octansäure, Decansäure,Pyrethrine, Pyrethroide, Methyl-nonyl-keton (Undecan-2-on), cyclo-Alkan-Carbonsäuren, Permethrin und (R)-p-mentha-1,8-diol, sowie wirksamen Derivaten der genannten Wirkstoffe, die aus der Literatur bekannt sind, oder Mischungen davon, wobei ein Repellent insbesondere bevorzugt ausgewählt ist aus N,N-Diethyl-3-methylbenzamid, 3-(Acetyl-butyl-amino)-propionsäure-ethylester und 1-(2-Methylpropyloxy-carbonyl)-2-(hydroxyethyl)-piperidin, (R)-p-mentha-1,8-diol oder Mischungen davon.

Bei den erfindungsgemäßen Zubereitungen, die Repellentien enthalten, handelt es sich dabei vorzugsweise um Insektenabwehrmittel. Insektenabwehrmittel werden vorzugsweise in Form von Lösungen, Gelen, Stiften, Rollern, Pump-Sprays und Aerosol-Sprays angeboten, wobei Lösungen und Sprays den Hauptteil der im Handel erhältlichen Produkte bilden. Basis für diese beiden Produktformen sind meist alkoholische bzw. wässrig-alkoholische Lösungen unter Zusatz fettender Substanzen und leichter Parfümierung. Es kann sich bei den erfindungsgemäßen Zubreitungen aber insbesondere auch um Emulsionen oder Gele handeln, die zusätzliche Wirkstoffe enthalten bzw. deren Hauptzweck in einer anderen Anwendung, beispielsweise dem Lichtschutz oder der Tagespflege besteht.

In einer bevorzugten Ausführungsform der vorliegenden Erfindung handelt es sich bei der einzusetzenden Zubereitung um eine Zubereitung zum Schutz von Körperzellen gegen oxidativen Stress, insbesondere zur Verringerung der Hautalterung, dadurch gekennzeichnet, dass sie neben den genannten Stoffen ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). \

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydroxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es

wird dort beobachtet, dass Dihydroxyflavone mit einer OH-Gruppe benachbart zur Ketofunktion oder OH-Gruppen in 3'4'-oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidanten Wirkung von Hydroxyflavonen. Über den gesamten pH-Bereich zeigt Quercetin die höchste Aktivität der untersuchten Strukturen.

Geeignete Antioxidantien sind weiter Verbindungen der Formel I

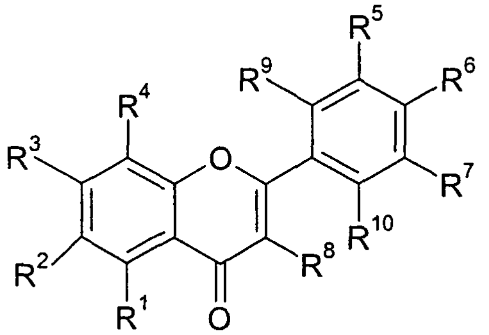

I

wobei $R^1$ bis $R^{10}$ gleich oder verschieden sein können und ausgewählt sind aus

- H
- $OR^{11}$
- geradkettigen oder verzweigten $C_1$- bis $C_{20}$-Alkylgruppen,
- geradkettigen oder verzweigten $C_3$- bis $C_{20}$-Alkenylgruppen,
- geradkettigen oder verzweigten $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können,
- wobei alle $OR^{11}$ unabhängig voneinander stehen für
- OH
- geradkettige oder verzweigte $C_1$- bis $C_{20}$-Alkyloxygruppen,
- geradkettigen oder verzweigten $C_3$- bis $C_{20}$-Alkenyloxygruppen,
- geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
- $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,
  mit der Maßgabe, dass mindestens 4 Reste aus $R^1$ bis $R^7$ stehen für OH und dass im Molekül mindestens 2 Paare benachbarter Gruppen - OH vorliegen,
- oder $R^2$, $R^5$ und $R^6$ für OH und die Reste $R^1$, $R^3$, $R^4$ und $R^{7-10}$ für H stehen,

wie sie in der Deutschen Patentanmeldung DE-A-10244282 beschrieben sind.

Erfindungsgemäß insbesondere bevorzugte Zubereitungen können auch dem Sonnenschutz dienen und enthalten dann auch UV-Filter.

Prinzipiell kommen alle UV-Filter für eine Kombination mit den erfindungsgemäß einzusetzenden Beugungsfarbmitteln in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),

Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan (z.B. Eusolex® 8020),

Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),

Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),

Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),

4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),

Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden. Diese organischen UV-Filter werden in der Regel in einer Menge von 0,5 bis 10 Gewichtsprozent, vorzugsweise 1 - 8 %, in den Formulierungen eingearbeitet.

Weitere geeignete organische UV-Filter sind z.B.

- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- $\alpha$-(Trimethylsilyl)-$\omega$-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] ($n \approx 60$) (CAS-Nr. 207 574-74-1 )
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),

Weitere geeignete UV-Filter sind auch Methoxyflavone entsprechend der älteren Deutschen Patentanmeldung DE-A-10232595.

Organische UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA, Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfonsäure sowie ihre Kalium-, Natrium- und Triethanol-aminsalze.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgenden Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind beispielsweise auch sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, $\alpha$-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), $\beta$-Mannosylglycerat (Firoin), $\beta$-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß der unten stehenden Formel II eingesetzt,

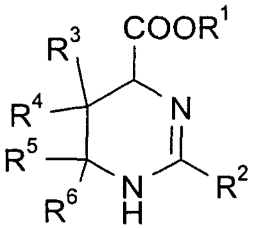

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Erfindungsgemäß insbesondere bevorzugt ist es dabei, wenn die kompatiblen Solute ausgewählt sind aus Di-myo-inositol-phosphat (DIP), cyclisches 2,3-diphosphoglycerat (cDPG), 1,1- Diglycerin-Phosphat (DGP), β-Mannosylglycerat (Firoin), β- Mannosylglyceramid (Firoin-A) oder/und Di-mannosyl-di-inositolphosphat (DMIP), Ectoin, Hydroxyectoin oder Mischungen davon.

Unter den ebenfalls bevorzugt eingesetzten Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

In einer weiteren ebenfalls bevorzugten Ausführungsform der vorliegenden Erfindung enthält die erfindungsgemäße Zubereitung mindestens einen Selbstbräuner.

Als vorteilhafte Selbstbräuner können unter anderem eingesetzt werden:

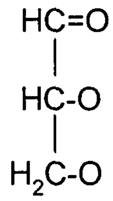
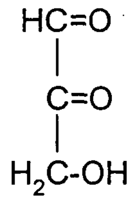
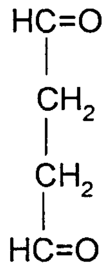
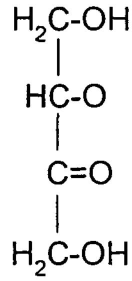

Glycerolaldehyd Hydroxymethylglyoxal γ-Dialdehyd Erythrulose

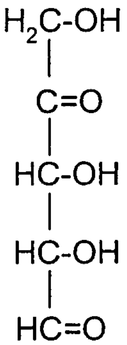
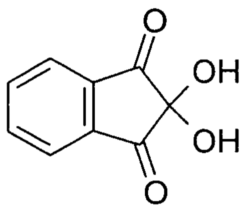

6-Aldo-D-Fructose Ninhydrin

Ferner ist das 5-Hydroxy-1,4-naphtochinon (Juglon) zu nennen, das aus den Schalen frischer Walnüsse extrahiert wird

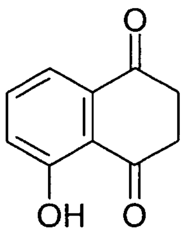

5-Hydroxy-1,4-naphtochinon (Juglon)

sowie das in den Henna-Blättern vorkommende 2-Hydroxy-1,4-naphtochinon (Lawson).

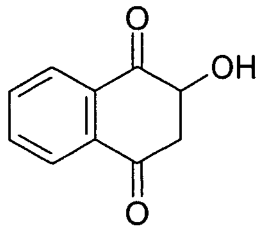

2-Hydroxy-1,4-naphtochinon (Lawson)

Ganz besonders bevorzugt ist das 1,3-Dihydroxyaceton (DHA), ein im menschlichen Körper vorkommender dreiwertiger Zucker und dessen Derivate.

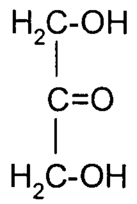

1,3-Dihydroxyaceton (DHA)

Alle Verbindungen oder Komponenten, die in den Zubereitungen verwendet werden können, sind entweder bekannt und käuflich erwerbbar oder können nach bekannten Verfahren synthetisiert werden.

Die Beugungsfarbmittel, Effektpigmente und weiteren Wirkstoffe können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere auch Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlange von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexyl-isostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

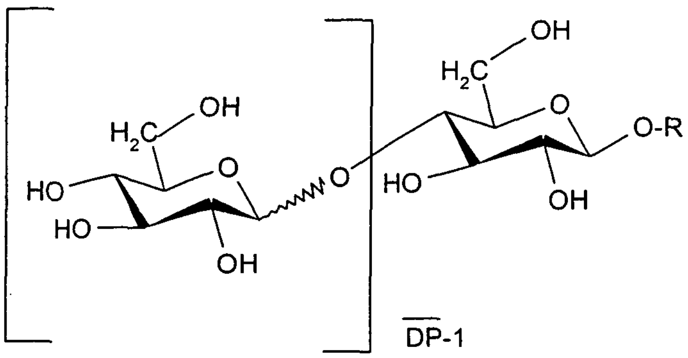

$\overline{DP}$-1

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \Sigma \frac{p_i}{100} \cdot i$$

Dabei stellen $p_1$, $p_2$, $p_3$ ... bzw. $p_i$ den Anteil der einfach, zweifach dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2-1,4, insbesondere von 1,3 gewählt.

Der Wert DP trägt den Umstande Rechnung, dass Alkylglucoside herstellungsbedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werder gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

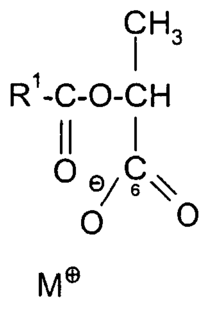

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

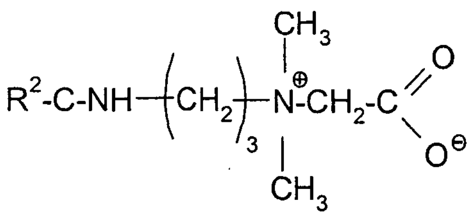

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsionen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulatoren verwendet werden. Es ist vorteilhaft, weitere übliche Co-emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18) stearylether (Steareth-18), Polyethylenglycol(19)stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)-isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20) -isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol(17) cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20), Polyethylenglycol(12) oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)-isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)-cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(1 5)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)gly-ceryllaurat, Polyethylenglycol(22)glyceryllaurat, Polyethylenglycol(23)gly-ceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbi-tanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder

Pigmente, die das Mittel selbst oder die Haare färben oder andere für die Haarpflege üblicherweise verwendete Ingredienzien.

Die erfindungsgemäßen Zubereitungen können dabei mit Hilfe von Techniken hergestellt werden, die dem Fachmann wohl bekannt sind.

Handelt es sich bei den erfindungsgemäß verwendeten Beugungsfarbmitteln um Filme, so ist eine bevorzugte Verwendung deren Einsatz als oder in künstlichen Nägeln. Ein weiterer Gegenstand der vorliegenden Anmeldung ist daher ein künstlicher Nagel enthaltend ein Beugungsfarbmittel. Dabei kann der Nagel aus dem Beugungsfarbmittelfilm bestehen oder das Beugungsfarbmittel kann die dekorative Schicht eines künstlichen Nagels bilden.

So kann beispielsweise eine Folie enthaltend Beugungsfarbmittel nach passendem Zuschnitt auf die individuelle Finger- bzw. Fußnagel-Größe, vom Trägerblatt abgelöst und auf den Finger/Fußnagel aufgebracht und anschließend mit klarem Nagellack fixiert werden. Alternativ können die Folien selbst mit einer klebenden Schicht versehen werden, so dass die nachträgliche Lackierung entfallen kann. Auch das Einlegen der Folie in einen Lack vor dem Aufbringen auf den Fingernagel ist eine Variante der vorliegenden Erfindung bei der nachträgliches Lackieren entfallen kann.

Weiter können die erfindungsgemäß einzusetzenden Beugungsfarbmittel auch in Lacken, wie UV-härtbaren Acrylharzen, die zur Herstellung künstlicher Nägel verwendet werden, eingesetzt werden.

Eine weitere erfindungsgemäß bevorzugte Verwendung der Beugungsfarbmittel ist der Einsatz in Nagelpflegemitteln, insbesondere Nagellacken. Dabei kann es insbesondere bevorzugt sein, wenn das Nagelpflegemittel nur die oben beschriebenen Kugeln oder Kern-Mantel-Partikel enthält, sich die photonische Struktur, welche das eigentliche Beugungsfarbmittel ausmacht, erst beim oder nach dem Auftragen des Lackes ausbildet. In einer anderen, ebenfalls bevorzugten Ausführungsform können die Nagelpflegemittel jedoch auch direkt Beugungsfarbmittel, insbesondere in pigmentärer Form, enthalten.

Nagelpflegemittel, insbesondere in Form von Nagellacken, gehören zu den am meisten verwendeten dekorativen Kosmetika. Sie enthalten meist ein synthetisches Harz als Filmbildner sowie anorganische oder organische Pigmente oder Farbstoffe. Die Nagellacke sollen hohen Glanz, hohe Härte und gute Haftung auf keratinhaltigen Substanzen, wie Fingernägeln zeigen und bei Raumtemperatur rasch zu einem nicht klebrigen gleichmäßigen Film trocknen. Der hohe Glanz und die gute Haftung sollen über einen möglichst langen Zeitraum erhalten bleiben. Damit die Nagellacke mit üblichen Nagellackentfernern wieder entfernt werden können, müssen die verwendeten filmbildenden Harze in Wasser-Aceton-Gemischen löslich sein. Dagegen sollen die filmbildenden Harze in Wasser oder Wasser-Alkohol- Gemischen unlöslich sein, damit der Nagellack bei Kontakt mit Wasser oder beim Umgang mit üblichen Haushaltschemikalien nicht angelöst wird.

Typische zum erfindungsgemäßen Einsatz geeignete Nagellacke bestehen im wesentlichen aus Lösungen, Suspensionen oder Emulsionen bestimmter Substanzen. Es sind hier insbesondere die Bindemittel, wie Nitrocellulose oder verschiedenen Kunstharze , wie insbesondere Polyacrylate, Polymethacrylate, Toluolsulfonamid-Formaldehyd-Harze, Toluolsulfonamid-Epoxid-Harze, Alkydharze oder Polyvinylacetat zu nennen. Weiter enthalten Nagellacke üblicherweise geeignete Lösungsmittel für die Bindemittel, wobei üblicherweise Lösemittelgemische eingesetzt werden. Typische Lösemittel sind neben Wasser Alkohole, Ester und Ketone, wie insbesondere Methyl-, Ethyl-, Propyl-, und Butylacetat, Isopropylalkohol und n-Butylalkohol. Die Nagellacke können ggf. mit weiteren Zusätzen zur Gestaltung des optischen Erscheinungsbildes, z. B. löslichen Farbstoffen versetzt sein. Zusätzlich können in der Mischung Weichmacher (wie z. B. Kampfer oder Dibutylphtalat) und Verschnittstoffe (z. B. Toluol oder Xylol) enthalten sein. Diese Nagellacke härten durch Verdunsten des Lösungsmittels sowie der anderen flüchtigen Bestandteile in Abhängigkeit von der Schichtdicke des Lackes, der Temperatur und der Art des Lösungsmittels aus. Daneben ist die Aushärtezeit noch von anderen Einflüssen (z. B. Luftanströmung) abhängig.

Erfindungsgemäß insbesondere bevorzugt sind dabei Nagelpflegemittel auf Wasserbasis, die vorzugsweise Polyurethane oder Polyacrylate als Bindemittel enthalten.

So beschreibt die EP-A-0 424 112 Nagellackformulierungen, die als Bindemittel ein Emulsionspolymerisat mit Kern/Schale-Aufbau enthalten. Das Polymer der äußeren Schale hat eine Erweichungstemperatur, die niedriger als die des Polymers der inneren Schale ist. Die DE-19727504 offenbart wässrige kosmetische Formulierungen, insbesondere Nagellackformulierungen, die als Bindemittel ein Emulsionspolymerisat enthalten, das durch Polymerisieren eines Gemisches bestimmter Monomere in Gegenwart eines Monomere mit einer ionischen oder ionogenen Gruppe enthaltenden Polymerisats erhältlich ist.

Wasserbasierte Nagellacke enthalten neben Wasser wasserlösliche Harze, wie beispielsweise Polyurethanharze, Acrylharze, Alkydharze, Epoxidharze oder Melaminharze. Dabei ist das Harz vorzugsweise so gewählt, dass es wasserlöslich ist, im Wasser weiß oder transparent erscheint, kostengünstig und einfach zu verarbeiten ist, möglichst nicht brennbar und nicht toxisch oder hyperallergen ist. Wesentlich kann weiter sein, dass der Lack schnell trocknend ist und gute filmbildende Eigenschaften bei Temperaturen ab 10°C aufweist.

In einer besonders bevorzugten Erfindungsvariante dient das Beugungsfarbmittel oder die Vorstufe für das Beugungsfarbmittel in dem Nagelpflegemittel gleichzeitig als Bindemittel. Dabei enthält das Nagelpflegemittel vorzugsweise kein weiteres Bindemittel und es handelt sich bei der Vorstufe für das Beugungsfarbmittel vorzugsweise um

Kern-Mantel-Partikel, wie sie bereits oben beschrieben wurden. Insbesondere bevorzugt handelt es sich dabei um Kern-Mantel-Partikel mit Polyacrylat-Mantel oder Polyurethan-Mantel. Entsprechende vorzugsweise einzusetzende Kern-Mantel-Partikel werden, wie bereits weiter oben ausgeführt, insbesondere in der Europäischen Patentanmeldung EP-A-0 955 323 bzw. der Internationalen Patentanmeldung WO 03/25035 beschrieben.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen. Die vollständige Offenbarung aller vor- und nachstehend aufgeführten Anmeldungen und Veröffentlichungen sind durch Bezugnahme in diese Anmeldung eingeführt. Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden. Es werden die INCI-Namen der verwendeten Rohstoffe angegeben (die INCI-Namen werden definitionsgemäß in Englischer Sprache angegeben).

**Beispiele**

Verwendete Abkürzungen:

| | |
|---|---|
| ALMA | Allylmethacrylat |
| AN | Acrylnitril |
| APS | Ammoniumperoxodisulfat |
| BDDA | Butan-1,4-dioldiacrylat |
| CHMA | Cyclohexylmethacrylat |
| MMA | Methylmethacrylat |
| SDS | Dodecylsulfat Natriumsalz |
| SDTH | Natriumdithionit |
| PCHMA | Poly(cyclohexylmethacrylat) |
| PS | Poly(Styrol) |

**Beispiel 1 Herstellung von Kugeln als primäre Bausteine**

**Beispiel 1a: Herstellung von monodispersen Siliciumdioxidkugeln eines Durchmessers von 250 nm**

In einem Kolben werden 1140 ml Ethanol und 430 ml VE-Wasser vorgelegt. Unter leichtem Rühren wird die Mischung auf 63°C temperiert. Dann werden 266 ml 25% Ammoniaklösung dazugegeben. Unter starkem Rühren werden (auf ebenfalls 63°C temperierte) 168 ml Tetraethylorthosilikat auf einmal und schnell dazugegeben. Nach 15 Sekunden Nachrühren wird der Rührer abgestellt und die Dispersion 18 h stehen gelassen. Nach Einengung der Dispersion erfolgt eine azeotrope Destillation, um das Ethanol und den Ammoniak zu entfernen, so dass sich die $SiO_2$-Kugeln in einer rein wässrigen Dispersion befinden.

**Beispiel 1b: Herstellung von monodispersen PMMA-Kugeln eines Durchmessers von 300 nm**

Ein Rundkolben wird mit 540 ml destilliertem Wasser und 100 ml Methylmethacrylat beschickt. Nach Aufheizen auf 80°C wird ein Stickstoffgaspolster auf die Dispersion gegeben. Der Impellerrührer wird auf 300 U/min eingestellt, um danach 0,5 g 2,2'-Azobis(2-methylpropionamidin)dihydrochlorid hinzuzugeben. Nach 2 h ist die Reaktion beendet und der Kolbeninhalt wird abgekühlt. Das Produkt wird durch mehrmaliges Zentrifugieren und Dekantieren gereinigt.

**Beispiel 2: Organisation der Kugeln in eine kolloidkristalline Packung (Opalpackung)**

Die Dispersionen aus Beispiel 1 a bzw. 1 b werden über einem Büchnertrichter mit feinem Filterpapier über Wasserstrahlpumpenvakuum abgesaugt. Zuerst gelangen die kolloiden Kugeln durch die Filternutsche hindurch (erkennbar am trüben Filtrat), es erfolgt aber schnell eine teilweise Verstopfung der Filterporen, so dass nur noch das Wasser abgesaugt wird und die Kugeln als Filterkuchen zurück bleiben. Die regelmäßige Anordnung der Kugeln erzeugt schillernde Farbeffekte.

**Beispiel 3: Herstellung von Opalfarbpartikeln**

Der ausgehend von Beispiel 1 a in Beispiel 2 aus $SiO_2$-Kolloidkugeln hergestellte, schillernde Filterkuchen wird vorsichtig mit dem Papierfilter in einem Ofen auf 400°C erhitzt zur Vernetzung der $SiO_2$-Kugeln. Nach Abkühlen wird das schillernde Material entnommen und vorsichtig zerkleinert. Es resultieren schillernde Opalfarbpartikel.

**Beispiel 4: Herstellung von inversen Opalfarbpartikeln**

Der ausgehend von Beispiel 1 b in Beispiel 2 aus PMMA-Kolloidkugeln hergestellte, schillernde Filterkuchen wird mit einer Precursorlösung, bestehend aus 7 ml Tetraethoxysilan, 4,7 ml Ethanol, 1 ml konz. HCl und 3 ml Wasser imprägniert unter schwacher Vakuumabsaugung. Der beladene Filterkuchen wird in einem Ofen auf 500°C erhitzt über einen Zeitraum von 10 h. Hierbei werden die PMMA-Kugeln pyrolytisch abgebaut und es bleiben schillernd farbige Pulver aus inversen Opalen aus $SiO_2$ zurück.

**Beispiel 5: Herstellung von Partikeln mit Kern aus Polystyrol, Zwischenschicht aus p(MMA-co-ALMA) und Mantel aus Poly(cyclohexylmethacrylat)**

In einem auf 75°C vorgeheizten 1-l-Rührkesselreaktor mit Propellerrührer, Argon-Schutzgaseinleitung und Rückflußkühler wird eine auf 4°C temperierte Vorlage, bestehend aus 217 g Wasser, 3,6 g Styrol (Fa. MERCK, entstabilisiert) und 130 mg SDS (Fa. MERCK) eingefüllt und unter starkem Rühren dispergiert. Direkt nach dem Einfüllen wird die Reaktion durch direkt aufeinanderfolgende Zugabe von 50 mg SDTH (Fa. MERCK), 350 mg APS (Fa. MERCK) und wiederum 50 mg SDTH, jeweils in 5g Wasser gelöst, gestartet. Nach 20 min wird eine Monomeremulsion aus 8,1 g BDDA (Fa. MERCK, entstabilisiert), 72,9 g Styrol (Fa. MERCK, entstabilisiert), 0,375 g SDS, 0,1 g KOH und 110 g Wasser über einen Zeitraum von 120 min kontinuierlich zudosiert. Der Reaktorinhalt wird 30 min ohne weitere Zugabe gerührt. Danach erfolgt eine Zugabe von 200 mg APS, gelöst in 5 g Wasser. Nach weiteren 60 min Rühren wird eine zweite Monomeremulsion aus 1,5 g ALMA (Fa. MERCK, entstabilisiert), 13,5 g MMA (Fa. MERCK, entstabilisiert), 0,075 g SDS und 20 g Wasser über einen Zeitraum von 25 min kontinuierlich zudosiert. Der Reaktorinhalt wird anschließend 30 min ohne weitere Zugabe gerührt. Danach erfolgt eine Zugabe von 200 mg APS, gelöst in 5 g Wasser. Es wird anschließend eine Monomeremulsion aus 120 g CHMA (Fa. Degussa, entstabilisiert), 120 g Wasser und 0,4 g SDS über einen Zeitraum von 150 min kontinuierlich zudosiert. Zur nahezu vollständigen Abreaktion der Monomere wird anschließend noch 60 min gerührt. Die Kern-Mantel-Partikel werden anschließend in 1 l Methanol ausgefällt, die Fällung durch Zugabe von 25 g konzentrierter wässriger Kochsalzlösung vervollständigt, die Suspension mit 1 l dest. Wasser versetzt, abgenutscht und getrocknet.

**Beispiel 6: Herstellung von Partikeln mit Kern aus Poly(methylmethacrylat) und Mantel aus Polystyrol**

In einem auf 75°C vorgeheizten 1-l-Rührkesselreaktor mit Propellerrührer, Argon-Schutzgaseinleitung und Rückflußkühler wird eine auf 4°C temperierte Vorlage, bestehend aus 217 g Wasser, 0,4 g ALMA (Fa. MERCK, entstabilisiert), 3,6 g MMA (Fa. MERCK, entstabilisiert) und 23 mg SDS (Fa. MERCK) eingefüllt und unter starkem Rühren dispergiert. Direkt nach dem Einfüllen wird die Reaktion durch direkt aufeinanderfolgende Zugabe von 30 mg SDTH (Fa. MERCK), 150 mg APS (Fa. MERCK) und wiederum 30 mg SDTH , jeweils in 5g Wasser gelöst, gestartet. Nach 20 min wird eine Monomeremulsion aus 9,6 g ALMA (Fa. Merck, entstabilisiert), 96 g MMA (Fa. MERCK, entstabilisiert), 0,35 g SDS, 0,1 g KOH und 130 g Wasser über einen Zeitraum von 120 min kontinuierlich zudosiert. Der Reaktorinhalt wird 60 min ohne weitere Zugabe gerührt.

Danach erfolgt eine Zugabe von 100 mg APS, gelöst in 5 g Wasser. Nach weiteren 10 min Rühren wird eine zweite Monomeremulsion aus 120 g Styrol (Fa. MERCK, entstabilisiert), 0,4 g SDS und 120 g Wasser über einen Zeitraum von 150 min kontinuierlich zudosiert. Zur nahezu vollständigen Abreaktion der Monomere wird anschließend noch 60 min gerührt. Die Kern-Mantel-Partikel werden anschließend in 1 l Methanol ausgefällt, die Fällung durch Zugabe von 25 g konzentrierter wässriger Kochsalzlösung vervollständigt, die Suspension mit 1 l dest. Wasser versetzt, abgenutscht und getrocknet.

**Beispiel 7: Herstellung von Partikeln durch isostatisches Verpressen und anschließendes Mahlen**

5 g des getrockneten Koagulates aus Beispiel 5 bzw. 6 werden in einem Mikroextruder (Fa. Thermo Haake, Minilab 5, Gleichläufer) mit 0,2 wt.-% Licolub Fa1 und 0,2 wt.-% Licolub WE 40 (beide von Fa. Clariant) bei einer Temperatur von 190°C und einer Schnecken-Drehzahl von 200 U/min über 30 min compoundiert. Das Extrudat aus der Lochdüse wird zu einer Schnecke aufgerollt und mit einer Laborpresse (Fa. Collin) zwischen zwei PET-Folien nach folgendem Programm zu dünnen Filmen verpresst:

| Schritt | 1 | 2 | 3 | 4 |
|---|---|---|---|---|
| Presszeit [min] | 3 | 3 | 1 | 8 |
| Druck [bar] | 100 | 250 | 250 | 200 |
| Temp. [C°] | 180 | 180 | 130 | 20 |

Die dünnen Filme werden dann in einem Achat-Mörser in kleine Partikel zermahlen.

**Beispiel 8: Herstellung einer Dispersion**

Es werden Formulierungen, bestehend aus jeweils 5 wt-% Beugungsfarbmittel aus Beispiel 3, 4 oder 7 in die thixotrope Nagellack Base 1348 (Fa. International Lacquers S.A.) durch Einrühren der Pigmentpartikel in den Lack hergestellt.
Die resultierende Dispersion wird auf eine Lackkarte aufgestrichen. Nach dem Trocknen und Aushärten der Lackformulierung resultiert eine Farbeffektbeschichtung aus hell reflektierenden Pigmenten, die je nach Betrachtungsrichtung eine intensiv grüne oder intensiv blaue Farbe aufweisen.

**Beispiel 9: Herstellung von Partikeln durch isostatisches Verpressen und anschließendes Mahlen**

Die Partikel aus Beispiel 8 werden analog zu Beispiel 7 zu Pigmenten verarbeitet.

**Beispiel 10: Herstellung von Kern-Mantel-Partikeln mit vernetzbarem Mantel**

In einem auf 75°C vorgeheizten Rührkesselreaktor mit Propellerrührer, Argon-Schutzgaseinleitung und Rückflußkühler wird eine auf 4°C temperierte Vorlage, bestehend aus 217 g Wasser, 0,4 g Butandioldiacrylat (Fa. Merck, entstabilisiert), 3,6 g Styrol (Fa. BASF, entstabilisiert) und 80 mg Natriumdodecylsulfat (SDS; Fa. Merck) eingefüllt und unter starkem Rühren dispergiert. Direkt nach dem Einfüllen wird die Reaktion durch direkt aufeinanderfolgende Zugabe von 50 mg Natriumdithionit (Fa. Merck), 250 mg Ammoniumperoxodisulfat (Fa. Merck) und wiederum 50 mg Natriumdithionit (Fa. Merck), jeweils in 5g Wasser gelöst, gestartet. Nach 10 min wird eine Monomeremulsion aus 6,6 g Butandioldiacrylat (Fa. Merck, entstabilisiert), 59,4 g Styrol (Fa. BASF, entstabilisiert), 0,3 g SDS, 0,1 g KOH und 90 g Wasser über einen Zeitraum von 210 min kontinuierlich zudosiert. Der Reaktorinhalt wird 30 min ohne weitere Zugabe gerührt. Anschließend wird eine zweite Monomeremulsion aus 3 g Allylmethacrylat (Fa. Merck, entstabilisiert), 27 g Methylmethacrylat (Fa. BASF, entstabilisiert), 0,15 g SDS (Fa. Merck) und 40 g Wasser über einen Zeitraum von 90 min kontinuierlich zudosiert. Der Reaktorinhalt wird anschließend 30 min ohne weitere Zugabe gerührt. Es wird anschließend eine Monomeremulsion aus 130 g Ethylacrylat (Fa. BASF, entstabilisiert), 139 g Wasser, 4 g Hydroxyethylmethacrylat und 0,33 g SDS (Fa. Merck) über einen Zeitraum von 180 min kontinuierlich zudosiert. Zur nahezu vollständigen Abreaktion der Monomere wird anschließend noch 60 min gerührt. Die Kern-Mantel-Partikel werden anschließend in 1 l Methanol ausgefällt, mit 1 l dest. Wasser versetzt, abgenutscht und getrocknet.

Beispiel 10a: Analog werden Kern-Mantel-Partikel hergestellt, deren Mantel aus 80 Gew.-% Cyclohexylmethacrylat, 18 Gew.-% Etylacrylat und 2 Gew.-% Hydroxyethylmethacrylat besteht.

**Beispiel 11: Herstellung von Partikeln durch Extrusion von Filmen und anschließendes Mahlen**

3 kg der Kern-Mantel-Partikel aus Beispiel 10 werden in einer Schneidmühle (Fa. Rapid, Typ: 1528) unter Eiskühlung zerkleinert und anschließend in einem Einschneckenextruder (Plasti-Corder; Fa. Brabender; Schneckendurchmesser 19 mm mit 1-Loch-Düse (3mm)) mit 2 Gew.-% Isocyanat-Härter (CRELAN™; Fa. Bayer) compoundiert. Nach einer Kühlstrecke wird in einem Granulator A 90-5 (Fa. Automatik) granuliert. Die Granulate werden auf einer Flachfolienanlage bestehend aus einem Einschneckenextruder (Fa. Göttfert; Typ: Extrusiometer; Schneckendurchmesser 20 mm; L/D 25), einem dickenverstellbaren Folienwerkzeug (Breite 135 mm) und einem temperierbaren Glättwerk (Fa. Leistritz; Walzendurchmesser 15 mm; Walzenbreite 350 mm) verarbeitet. Es wird ein Folienband von 125 mm Breite und 1 mm Dicke erhalten. Anschließend wird die Folie auf 190°C erhitzt. Nach erfolgter Vernetzung wird die spröde Folie in einem Walzwerk zu Pigment-Partikeln zerkleinert

**Beispiel 11a: Herstellung von Partikeln durch isostatisches Verpressen und anschließendes Mahlen**

Die Partikel aus Beispiel 10a werden analog zu Beispiel 7 zu Pigmenten verarbeitet.

**Beispiel 12: Herstellung von Opalfarbpartikeln**

Der ausgehend von Beispiel 1 a in Beispiel 2 aus $SiO_2$-Kolloidkugeln hergestellte, schillernde Filterkuchen wird vorsichtig mit einem geschmolzenen Wachs (Canaubawachs) getränkt. Nach Abkühlen wird das schillernde Material entnommen und vorsichtig zerkleinert. Es resultieren schillernde Opalfarbpartikel.

**Beispiel 13: Herstellung von Opalfarbpartikeln**

Der ausgehend von Beispiel 1 b in Beispiel 2 aus PMMA-Kolloidkugeln hergestellte, schillernde Filterkuchen wird vorsichtig mit einem geschmolzenen Lanolin (Satulan®; CRODA OLEO CHEMICALS LTD) getränkt. Nach Abkühlen wird das schillernde Material entnommen und vorsichtig zerkleinert. Es resultieren schillernde Opalfarbpartikel.

**Beispiel 14: Herstellung von inversen Opalfarbpartikeln durch Ätzen**

**Beispiel 14a: Funktionalisierung der $SiO_2$-Kerne aus Beispiel 1**

Zu 1,3 ℓ ethanolischer Suspension mit 2,5 Gew.-% $SiO_2$ ($SiO_2$-Suspension mit violetter Trocknungsfarbe (Wellenlängenmaximum I111 = 400 nm, mittlerer Teilchendurchmesser nach TEM 201 nm; nach Beispiel 1), 0,69 M $NH_3$ und 2 M $H_2O$ werden unter Rühren bei Raumtemperatur 3 mℓ MPS gelöst in Ethanol zugegeben. Die Mischung wird am Rotationsverdampfer zunächst unter Normaldruck langsam auf 65°C erwärmt. Nach 1,5 h wird durch Verringern des Drucks die Destillation eines azeotropen Gemisches aus Ethanol und Wasser gestartet. Die abdestillierte Flüssigkeit wird durch absolutes Ethanol ersetzt. Insgesamt werden 1,2 ℓ Ethanol-WasserGemisch entfernt. Nach 2 h wird die Reaktionslösung auf 300 ml eingeengt und in einen 1 ℓ Rundkolben umgefüllt. Es werden 0,06 g SDS, gelöst in 120 g Wasser zugegeben und erneut bei 65°C Ethanol abdestilliert. Die abdestillierte Flüssigkeit wird durch Wasser ersetzt.

**Beispiel 14b: Emulsionspolymerisation**

Die Emulsionspolymerisation wird in einen doppelwandigen, auf 75°C thermostatisierten 250 mℓ Glasreaktor mit Inertgaszuführung, Propellerrührer und Rückflusskühler durchgeführt. 110 g (enthalten 17 g $SiO_2$) $SiO_2$-Suspension nach Beispiel 2 werden 20 min mit Argon durchperlt. Dann werden 0,1 g SDS zugegeben und die Mischung im Reaktor vorgelegt. Anschließend werden 0,05 g SPS, gelöst in 3 g Wasser, zugegeben. Nach 15 min wird eine Monomeremulsion aus 5,4 g MMA, 0,6 g ALMA, 0,02 g SDS (0,33 Gew.-% auf Monomer), 0,04 g KOH und 30 g Wasser über einen Zeitraum von 90 min kontinuierlich zudosiert. Der Reaktorinhalt wird 20 min ohne weitere Zugabe gerührt. Danach erfolgte eine Zugabe von 0,02 g APS, gelöst in 3 g Wasser. Nach 10 min wird eine zweite Monomeremulsion aus 20 g CHMA, 0,08 g SDS (0,4 Gew.-% auf Monomer) und 40 g Wasser über einen Zeitraum von 200 min kontinuierlich zudosiert. Zur nahezu vollständigen Abreaktion der Monomeren wird anschließend noch 120 min gerührt. Die Kern-Mantel-Partikel werden anschließend in 500 mℓ Ethanol ausgefällt, die Fällung durch Zugabe von 15 g konzentrierter wässriger Kochsalzlösung vervollständigt, die Suspension mit 500 mℓ dest. Wasser versetzt, abgenutscht und das Polymere bei 50°C im Vakuum getrocknet.

**Beispiel 14c Herstellung des Templatfilmes**

Die getrockneten, pulverförmigen Polymere aus Beispiel 3 werden im Extruder (Mikroextruder der DSM Research) bei 200°C granuliert. Die Granulate werden in einer hydraulischen Presse (Collin 300 P) erwärmt und bei einem vorgegebenen Hydraulikdruck verpresst. Als Form werden plane, mit PET-Folie bedeckte Metallplatten verwendet. Ein typisches Pressprogramm zur Herstellung von Filmen mit einem Durchmesser von etwa 10 cm und einer Dicke von etwa 0,15 mm ist:

Einwaage 2 - 3 g Polymer;
Vorwärmen 5 min bei 180°C, drucklos;
3 min Pressen mit 1 bar Hydraulikdruck bei 180°C;
3 min Pressen mit 150 bar Hydraulikdruck bei 180°C;
10 min langsames Kühlen bei 150 bar Hydraulikdruck, erreicht werden etwa 90°C;
schnelles Abkühlen auf Raumtemperatur, drucklos.

**Beispiel 14d: Ätzen der Filme mit Flusssäure**

Die Filme werden in offenen Gefäßen mit Flußsäure (10 Gew.%) überschichtet und eine Woche bei RT belastet. Verdunstende Flußsäure wird durch frische ersetzt. Nach Spülen mit Wasser und Trocknen zeigen die geätzten Filmstücke deutlich erkennbare Reflexionsfarben.
Die Untersuchung von Ultradünnschnitten (100 nm) der Filme nach dem Ätzen belegt, dass unter Erhalt der Ordnung die $SiO_2$-Kerne aus den Filmen herausgelöst werden und geordnet poröse Filme entstanden sind.

**Beispiel 14e: Mahlen der porösen Filme**

Durch Mahlen der geordnet porösen Filme werden schillernde inverse Opalpulver erhalten.

**Beispiel 15: Herstellung von inversen Opalfarbpartikeln durch UV-Abbau**

**Herstellung eines Latex PTBMAcsPS**

Zu einer auf 4°C temperierten Emulsion, bestehend aus 217 g Wasser, 0,4 g ALMA, 3,6 g TBMA und 30 mg SDS werden 50 mg Natriumbisulfit, gelöst in 5g Wasser, zugemischt und die Emulsion in den auf 75°C vorgeheizten Reaktor überführt. Direkt nach dem Einfüllen wird die Reaktion durch Zugabe von 220 mg Natriumperoxodisulfat und weiteren 50 mg Natriumbisulfit, jeweils in 5g Wasser gelöst, gestartet. Nach 20 min wird Monomeremulsion I aus 9,6 g ALMA, 96 g TBMA, 0,45 g SDS, 0,1 g KOH und 130 g Wasser über einen Zeitraum von 180 min kontinuierlich zudosiert. Der Reaktorinhalt wird 30 min ohne weitere Zugabe gerührt. Danach erfolgt eine Zugabe von 150 mg Natriumperoxodisulfat, gelöst in 5 g Wasser. Nach 15 min Rühren wird Monomeremulsion II aus 120g Styrol, 0,4 g SDS und 120 g Wasser über einen Zeitraum von 200 min kontinuierlich zudosiert. Zur nahezu vollständigen Abreaktion der Monomeren wird anschließend noch 60 min gerührt. Getrocknete Proben des Latex zeigen eine grüne Farbe. Die elektronenmikroskopische Untersuchung von Niederschlägen des Latex zeigt, dass die Polymerpartikeln eine unregelmäßige Form und eine durchschnittliche Partikelgröße von etwa 210 nm aufweisen. Die Kern-Mantel-Partikel werden anschließend in 1 Liter Ethanol ausgefällt, die Fällung durch Zugabe von 25 g konzentrierter wässriger Kochsalzlösung vervollständigt, die Suspension mit 1 Liter dest. Wasser versetzt, abgenutscht und das Polymere bei 50°C im Vakuum getrocknet.

Durch Veränderung von Emulgatorart und -konzentration werden weitere Latices mit größerem Partikeldurchmesser und gleichmäßigerer Partikelform hergestellt:

| Beispiel | 15a | 15b | 15c | 15d |
|---|---|---|---|---|
| **Vorlage** | | | | |
| Wasser | 217 g | 217 g | 217 g | 217 g |
| ALMA | 0,4 g | 0,4 g | 0,4 g | 0,4 g |
| TBMA | 3,6 g | 3,6 g | 3,6 g | 3,6 g |
| SDS | 0,03 g | 0,03 g | 0,03 g | 0,03 g |
| **Start** | | | | |
| Natriumbisulfit | 0,05 g | 0,05 g | 0,05 g | 0,05 g |
| Natriumperoxo disulfat | 0,22 g | 0,2 g | 0,2 g | 0,2 g |
| Natriumbisulfit | 0,05 g | 0,05 g | 0,05 g | 0,05 g |
| **Emulsion I** | | | | |
| Wasser | 130 g | 130 g | 130 g | 130 g |
| TBMA | 96 g | 96 g | 96 g | 96 g |
| ALMA | 9,6 g | 9,6 g | 9,6 g | 9,6 g |
| SDS | 0,45 g | 0,4 g | 0,4 g | 0,4 g |
| Triton X 405 | | 0,2 g | 0,2 g | 0,2 g |
| KOH | 0,1 g | 0,1 g | 0,1 g | 0,1 g |

Tabelle fortgesetzt

| | | | | |
|---|---|---|---|---|
| **Initiator**<br>Natriumperoxo disulfat | 0,15 g | 0,1 g | 0,1 g | 0,1 g |
| **Emulsion II**<br>Wasser | 120 g | 140 g | 140 g | 140 g |
| Styrol<br>SDS<br>Triton X405 | 120 g<br>0,4 g | 120 g<br>0,4 g<br>0,3 g | 120 g<br>0,4 g<br>0,3 g | 120 g<br>0,4 g<br>0,3 g |
| **Farbe** | grün | rot (Nebenebenen) | grün (Nebenebenen) | rötlich |

**Pressen der Filme**

Zur Herstellung von Filmen werden die Polymerpulver aus den Beispielen 15a-15d in einer hydraulischen Presse (Collin 300 P) erwärmt und die Schmelze bei einem vorgegebenen Hydraulikdruck gepresst. Als Form werden plane, mit PET-Folie bedeckte Metallplatten verwendet. Ein typisches Pressprogramm zur Herstellung von Filmen mit einem Durchmesser von etwa 10 cm und einer Dicke von etwa 0,2 mm ist:

Einwaage 1 - 2 g Polymer;
Vorwärmen 5 min bei 180°C, drucklos;
3 min Pressen mit 1 bar Hydraulikdruck bei 180°C;
3 min Pressen mit 150 bar Hydraulikdruck bei 180°C;
10 min langsames Kühlen bei 150 bar Hydraulikdruck, erreicht werden etwa 90°C;
schnelles Abkühlen auf Raumtemperatur, drucklos.

**Herstellung von Formkörpern mit homogenen, regelmäßig angeordneten Kavitäten**

Die Filme werden über einen Zeitraum von 24 h unter eine UV-Lampe (Hochdruck HG-Dampflampe, Leistung 300 Watt, Abstand Lampe - Film: 20 cm) gelegt. Nach der UV-Beiastung zeigen die Filme brillante, irisierende Farbeffekte.

**Mahlen der porösen Filme**

Durch Mahlen der geordnet porösen Filme werden schillernde inverse Opalpulver erhalten.

**Beispiel 16: Herstellung von inversen Opalfarbpartikeln durch Calcinieren**

In einem auf 75 °C temperierten 5 l Doppelmantelreaktor mit Doppelpropellerrührer, Argon-Schutzgaseinleitung und Rückflußkühler wird eine auf 4 °C temperierte Vorlage, bestehend aus 1519 g VE-Wasser, 2,8 g 1,4-Butandioldiacrylat (Fa. MERCK), 25,2 g Styrol (Fa. MERCK) und 1030 mg Natriumdodecylsulfat (Fa. MERCK) eingefüllt und unter starkem Rühren dispergiert.
Direkt danach wird die Reaktion durch aufeinanderfolgendes Einspritzen von 350 mg Natriumdithionit (Fa. MERCK), 1,75 g Ammoniumperoxodisulfat (Fa. MERCK) und wiederum 350 mg Natriumdithionit (Fa. MERCK), jeweils in ca. 20 ml Wasser gelöst, gestartet. Das Einspritzen erfolgt mittels Einwegspritzen.
Nach 20 min wird eine Monomeremulsion, bestehend aus 56,7 g 1,4-Butandioldiacrylat (Fa. MERCK), 510,3 g Styrol (Fa. MERCK), 2,625 g Natriumdodecylsulfat (Fa. MERCK), 0,7 g KOH und 770 g Wasser über einen Zeitraum von 120 min kontinuierlich über die Taumelkolbenpumpe zudosiert.
Der Reaktorinhalt wird 30 min ohne weitere Zugabe gerührt.
Anschließend wird eine zweite Monomeremulsion, bestehend aus 10,5 g Allylmethacrylat (Fa. MERCK), 94,50 g Methylmetacrylat (Fa. MERCK), 0,525 g Natriumdodecylsulfat (Fa. MERCK) und 140 g Wasser über einen Zeitraum von 30 min über die Taumelkolbenpumpe kontinuierlich zudosiert. Nach ca. 15 min werden 350 mg Ammoniumperoxodisulfat (Fa. MERCK) zugegeben und danach noch 15 min gerührt.

Schließlich wird eine dritte Monomeremulsion, bestehend aus 200 g Ethylacrylat (Fa. MERCK), 0,550 g Natriumdodecylsulfat (Fa. MERCK) und 900 g Wasser über einen Zeitraum von 240 min kontinuierlich über die Taumelkolbenpumpe zudosiert. Anschließend wird 120 min nachgerührt. Vor und nach jedem Einleiten von Monomerenemulsionen und nach Einfüllen der Vorlage wird ca. eine Minute Argon als Schutzgaspolster in den Doppelmantelreaktor eingeleitet. Am nächsten Tag wird der Reaktor auf 95 °C erwärmt und eine Wasserdampfdestillation durchgeführt, um restliche,

nicht abreagierte Monomere aus der Latexdispersion zu entfernen.

Es resultiert eine Dispersion von Kern-Mantel-Partikeln, bei denen der Mantel einen Gewichtsanteil von ca. 22% hat. Der Kern aus Polystyrol ist vernetzt, die Zwischenschicht ist ebenfalls vernetzt ( p(MMA-co-ALMA)) und dient zum Pfropfen des Mantels aus unvernetztem Ehtylacrylat.

Zur Bildung der templatisierenden Struktur, d. h. der Organisation der Kern-Mantel-Partikel in eine dichte Kugelpackung, werden 5 g der Latexdispersion in eine flache Glasschale eines Durchmessers von 7 cm gegossen und an der Luft getrocknet, wobei bunt schillernde Flitter entstehen.

Ein solcher Flitter wird in einem Rundkolben mit der Öldrehschieberpumpe evakuiert. Anschließend wird eine Precursor-Lösung, bestehend aus 5 ml Tetra-n-butylorthotitanat in 5 ml absolutem Ethanol im statischen Vakuum dazugegeben, so dass der gelöste Precursor, getrieben von Kapillarkräften, in die Kavitäten des Templates eindringen kann. Über die Lösung, in dem sich das imprägnierte Template befindet, wird ein Argon-Polster gegeben. Diese Anordnung wird statisch über einige Stunden belassen, bevor im Argon Schutzgasstrom der imprägnierte Flitter entnommen und in einem Korund-Schiffchen im Rohrofen bei 500 °C calziniert wird. Es resultieren opaleszierende Flitter.

**Beispiel 17: Herstellung von inversen Opalfarbpartikeln durch Pyrolyse von Polymerfilmen**

**Herstellung der Kern-Mantel-Latices PMMA-PSAN$_{50}$ (Mantel aus 50 Gew. -% Styrol und 50 Gew-% Acrylnitril)**

In einer auf 4°C temperierten Vorlagen-Emulsion, bestehend aus 217 g Wasser, 0,4 g Allylmethacrylat (ALMA, Fa. MERCK), 3,6 g Methylmethacrylat (MMA, Fa. MERCK) und 20,5 mg Natriumdodecylsulfat (SDS, Fa. MERCK) werden 30 mg Natriumdithionit (SDTH, Fa. MERCK), gelöst in 5g Wasser, zugemischt.

Die Emulsion wird in einen auf 75°C temperierten 1-I-Doppelmantelrührkessel mit Rückflußkühler, Argongaseinleitung und Doppelpropellerrührer überführt.

Direkt nach dem Einfüllen der Emulsion wird die Reaktion durch Zugabe von 150 mg Ammoniumperoxodisulfat (APS, Fa. MERCK) und weiteren 30 mg Natriumdithionit (SDTH, Fa. MERCK), jeweils in 5g Wasser gelöst, gestartet.

Nach 20 min wird eine Monomeremulsion, bestehend aus 9,6 g ALMA (Fa. MERCK), 96 g MMA (Fa. MERCK), 0,35 g SDS (Fa. MERCK), 0,1 g KOH (Fa. MERCK) und 130 g Wasser über einen Zeitraum von 120 min kontinuierlich über eine Taumelkolbenpumpe zudosiert.

Der Reaktorinhalt wird 60 min ohne weitere Zugabe gerührt. Danach erfolgt eine Zugabe von 100 mg APS (Fa. MERCK), gelöst in 5 g Wasser.

Nach weiteren 10 min Rühren wird eine zweite Monomeremulsion, bestehend aus 60 g Styrol (Fa. MERCK), 60 g Acrylnitril, 0,33 g SDS (Fa. MERCK) und 120 g Wasser über einen Zeitraum von 160 min kontinuierlich über eine Taumelkolbenpumpe zudosiert.

Zur nahezu vollständigen Abreaktion der Monomere wird anschließend noch 60 min gerührt.

Die Kern-Mantel-Partikel werden anschließend in 1l Methanol koaguliert, die Fällung durch Zugabe von 25 g konzentrierter wässriger Kochsalzlösung vervollständigt, die Suspension mit 1 l dest. Wasser versetzt, abgenutscht und das polymere Koagulat bei 50°C im Vakuum getrocknet.

Mit Hilfe eines Transmissionselektronenmikroskops wird eine mittlere Teilchengröße der Partikel von 263 nm bestimmt.

**Herstellung der Kern-Mantel-Latices PMMA-PSAN$_{70}$ (Mantel aus 30 wt-% Styrol und 70 wt-% Acrylnitril)**

Rezeptur siehe oben, mit den folgenden Abweichungen:

Die Vorlagen-Emulsion enthält 22 mg SDS (Fa. MERCK),

die zweite Monomerenemulsion besteht aus 36 g Styrol (Fa. MERCK), 84 g Acrylnitril, 120 g Wasser, 0,4 g SDS (Fa. MERCK) und 0,34 g Triton X405™.

**Weiterverarbeitung des Koagulats in Filme**

Das Koagulat, bestehend aus PMMA-PSAN$_{50}$-Latexpartikeln wird in einem DSM-Mikroextruder bei 220 °C in Stickstoffatmosphäre zu einem Polymerstrang verarbeitet, der in 5 mm lange Granulate zerschnitten wird. Die Granulate werden zu Filmen verpresst.

Das Verpressen von jeweils 1 - 2 g Koagulat bzw. Granulat in Filme erfolgt bei folgenden Bedingungen in einer Collin 300 P-Laborpresse:

- Vorwärmen 5 min bei 180°C, drucklos;
- 3 min Pressen mit 1 bar bei 180°C;
- 3 min Pressen mit 150 bar bei 180°C;
- 10 min langsames Kühlen bei 150 bar, erreicht werden etwa 90°C;

- schnelles Abkühlen auf Raumtemperatur, drucklos.

Die erhaltenen Filme haben eine Stärke von ca. 0,2 mm, weisen eine winkelabhängige, bei senkrechter Aufsicht gelb-grüne Farbe auf und sind zähelastisch.

**Pyrolyse der Filme**

**Variante a:**

Die Filme werden in Luftatmosphäre bei 240°C im Muffelofen über 5 h pyrolysiert.
Die pyrolysierten Filme haben eine schwarze Grundfarbe, der eine violette Reflektionsfarbe bei senkrechter Aufsicht überlagert ist.

**Variante b:**

Die Filme werden in Luftatmosphäre bei 200 °C über 2 Wochen getempert. Die getemperten Filme, in denen die Polymerkerne noch vorhanden sind, haben eine braune Grundfarbe, der eine grüne Reflektionsfarbe bei senkrechter Aufsicht überlagert ist.
Die Filme werden anschließend in Luftatmosphäre bei 240°C im Muffelofen über 5 h pyrolysiert.
Die pyrolysierten Filme haben eine schwarze Grundfarbe, der eine violette Reflektionsfarbe bei senkrechter Aufsicht überlagert ist. Die Poren im Film haben eine nahezu sphärische Gestalt.

**Mahlen der porösen Filme**

Durch Mahlen der geordnet porösen Filme werden schillernde inverse Opalpulver erhalten.

**Formulierungsbeispiele**

In den folgenden Formulierungsbeispielen werden als Pigment jeweils die photonischen Effektmaterialien ausgewählt aus einem oder mehreren der vorstehenden Beispiele 3, 4, 7, 9, 11, 12, 13, 14, 15, 16 und 17. eingesetzt.
In den Formulierungen wird jeweils der Handelsname, die INCI-Bezeichnung, sowie der Anteil in Gew.-% und eine Bezugsquelle für die Rohstoffe angegeben.

**Beispiel A: Wet Lip Balm mit 10 % Beugungsfarbmittel**

| | | | |
|---|---|---|---|
| Phase A | | | |
| Pigment | | 10,00 % | (1) |
| Phase B | | | |
| Vegelatum Equiline EU103 | | 11,00 % | (2) |
| Candelilla Wax | Candelilla Cera (Candelilla Wax) | 7,00 % | (3) |
| Bienenwachs gebleicht | Cera Alba (Beeswax) | 5,00 % | (1) |
| Myritol 331 | Cocoglycerides | 8,00 % | (4) |
| Ozokerite Wax White # 77W | Ozokerite | 3,00 % | (3) |
| Isopropylmyristat | Isopropyl Myristate | 5,00 % | (4) |
| Eusolex® 2292 | Ethylhexyl Methoxycinnamate, BHT | 7,00 % | (1) |
| Eusolex® OCR | Octocrylene | 4,00 % | (1) |
| Oxynex® K flüssig | PEG-8, Tocopherol, Ascorbyl Palmitate, Ascorbic Acid, Citric Acid | 0,10 % | (1) |
| Propyl-4-hydroxybenzoat | Propylparaben | 0,10 % | (1) |
| Farbdispersion in Rizinusöl | | 0,50 % | |
| Rizinusöl | Ricinus Communis (Castor Oil) | 38,80 % | (5) |

Tabelle fortgesetzt

| | | | |
|---|---|---|---|
| Phase C | | | |
| Parfümöl Tendresse # 75418C | Parfum | 0,50 % | (6) |

Herstellung:

Die Bestandteile der Phase B werden unter Rühren auf 75 °C erhitzt bis alles geschmolzen ist. Phase A zugeben und gut durchrühren. Die Lippenstiftmasse dann in der auf 65 °C temperierten Gießapparatur füllen, Phase C zugeben und 15 Minuten rühren. Die homogene Schmelze wird in die auf 55 °C vorgewärmten Gießform gegossen.

Anschließend kühlt man die Formen ab und entfernt die Gießlinge kalt.
Nach Erwärmen der Lippenstifte auf Raumtemperatur werden die Lippenstifte kurz abgeflammt.

Bezugsquellen:

(1) Merck KGaA/Rona®
(2) Natunola Health Inc.
(3) Ross Waxes
(4) Cognis GmbH
(5) Henry Lamotte GmbH
(6) Haarmann & Reimer GmbH

**Beispiel B: Duschgel**

Phase A

| | | | |
|---|---|---|---|
| Pigment | | 0,10 % | (1) |
| Keltrol T | Xanthan Gum | 0,75 % | (2) |
| Wasser, demineralisiert | Aqua (Water) | 64,95 % | |

Phase B

| | | | |
|---|---|---|---|
| Plantacare 2000 UP | Decyl Glucoside | 20,00 % | (3) |
| Texapon ASV 50 | Sodium Laureth Sulfate, SodiumLaureth-8 Sulfate, Magnesium LaurethSulfate, Magnesium Laureth-8 Sulfate, Sodium Oleth Sulfate, Magnesium OlethSulfate | 3,60 % | (3) |
| Bronidox L | Propylene Glycol, 5-Bromo-5-Nitro-1,3-Dioxane | 0,20 % | (3) |
| Parfümöl Everest 79658 SB | Parfum | 0,05 % | (4) |
| 1 % FD&C Blue No. 1 in Wasser | Aqua (Water), CI 42090 (FD&C Blue No. 1) | 0,20 % | (5) |

Phase C

| | | | |
|---|---|---|---|
| Citronensäure Monohydrat | Citric Acid | 0,15 % | (1) |
| Wasser, demineralisiert | Aqua (Water) | 10,00 % | |

Herstellung:

Für Phase A wird das erfindungsgemäße Pigment in Wasser einrührt. Keltrol T unter Rühren langsam einstreuen und rühren bis es gelöst ist. Die Phasen B und C nacheinander hinzufügen und dabei langsam rühren bis alles homogen verteilt ist. Den pH-Wert auf 6,0 bis 6,5 einstellen.

Bezugsquellen:

(1) Merck KGaA/Rona®
(2) Kelco
(3) Cognis GmbH
(4) Haarmann & Reimer GmbH
(5) BASF AG

Beispiel C: Lidschatten

| | | | |
|---|---|---|---|
| Phase A | | | |
| Pigment | | 55,0 % | (1) |
| Biron® B 50 | CI 77163 (Bismuth Oxychloride) | 3,00 % | (1) |
| Colorona® Dark Blue | Perlglanzpigment: Mica, CI 77891 (Titanium Dioxide), CI 77510 (Ferric Ferrocyanide) | 10,00 % | (1) |
| Magnesiumstearat | Magnesium Stearate | 2,50 % | (1) |
| Weißer Ton (gestrichen) | Kaolin | 5,00 % | (1) |
| Hubersorb 600 | Calcium Silicate | 0,50 % | (2) |
| Talkum | Talc | 11,00 % | (1) |

| | | | |
|---|---|---|---|
| Phase B | | | |
| Amerchol L 101 | Lanolin Alcohol, Paraffinum Liquidum (Mineral Oil) | 10,70% | (3) |
| Super Hartolan | Lanolin Alcohol | 1,00 % | (4) |
| Ewalin 1751 | Petrolatum | 1,00 % | (5) |
| Propyl-4-hydroxybenzoat | Propylparaben | 0,10 % | (1) |
| Parfümöl Elegance # 79228 D MD | Parfum | 0,20 % | (6) |

Herstellung:

Bestandteile der Phase A zusammengeben und vormischen. Anschließend die Pudermischung unter Rühren tropfenweise mit der geschmolzenen Phase B versetzen. Die Puder werden bei 40 - 50 bar gepresst.

Bezugsquellen:

(1) Merck KGaA/Rona®
(2) J. M. Huber Corp.
(3) Amerchol
(4) Croda GmbH
(5) H. Erhard Wagner GmbH
(6) Haarmann & Reimer GmbH

Beispiel D: Eye Shadow Gel

| | | | |
|---|---|---|---|
| Phase A | | | |
| Pigment | | 15,00 % | (1) |
| Mica Black | CI 77499 (Iron Oxides), Mica, CI 77891 (Titanium Dioxide) | 5,00 % | (1) |
| Ronasphere® | Silica | 3,00 % | (1) |
| Carbopol ETD 2001 | Carbomer | 3,00 % | (2) |

Tabelle fortgesetzt

| | | | |
|---|---|---|---|
| Phase A | | | |
| Wasser, demineralisiert | Aqua (Water) | 60,00 % | |

| | | | |
|---|---|---|---|
| Phase B | | | |
| Glycerin, wasserfrei | Glycerin | 2,00 % | (1) |
| Germaben II | Propylene Glycol, Diazolidinyl Urea, Methylparaben, Propylparaben | 0,20 % | (3) |
| Triethanolamin reinst | Triethanolamine | 0,70 % | (1) |
| Wasser, demineralisiert | Aqua (Water) | 13,80 % | |

Herstellung:

Pigment und Ronasphere® werden im Wasser der Phase A dispergiert. Mit einigen Tropfen Citronensäure ansäuern um die Viskosität zu vermindern, Carbopol unter Rühren einstreuen. Nach vollständiger Lösung die vorgelöste Phase B langsam einrühren.

Bezugsquellen:

(1) Merck KGaA
(2) BF Goodrich GmbH
(3) ISP Global Technologies

**Beispiel E: Lip Gloss**

| | | |
|---|---|---|
| Phase A | | |
| Pigment | 10,00% | (1) |

| | | | |
|---|---|---|---|
| Phase B | | | |
| Indopol H 100 | Polybutene | 59,90 % | (2) |
| Bentone Gel MIO V | Quaternium-18 Hectorite, Propylene Carbonate, Paraffinum Liquidum (Mineral Oil) | 20,00 % | (3) |
| Eutanol G | Octyldodecanol | 6,00 % | (4) |
| RonaCareTM Tocopherolacetat | Tocopheryl Acetate | 1,00 % | (1) |
| Dow Coming 1403 Fluid | Dimethiconol, Dimethicone | 3,00 % | (5) |
| Propyl-4-hydroxybenzoat | Propylparaben | 0,05 % | (1) |
| Rouge Covapate W 3773 | Ricinus Communis (Castor Oil), CI 15850 (D&C) Red No. 6) | 0,05 % | (6) |

Herstellung:

Alle Bestandteile der Phase B werden zusammen eingewogen, auf 70 °C erhitzt und gut durchgerührt bis eine homogene Masse entstanden ist.
Dann werden die Pigmente zugegeben und nochmals durchgerührt. Die homogene Mischung füllt man bei 50 - 60 °C ab.

Bezugsquellen:

(1) Merck KGaA/Rona®
(2) BP Amoco
(3) Elementis Specialites
(4) Cognis GmbH

(5) Dow Corning
(6) Les Colorants Wackherr

**Beispiel F: Lidschatten - Kompaktpuder**

| | | | |
|---|---|---|---|
| Phase A | | | |
| Pigment | | 25,00 % | (1) |
| Colorona® Dark Blue | Mica, Ci 77891 (Titanium Dioxide), CI 77510 (Ferric Ferrocyanide) | 5,00 % | (1) |
| Talkum | Talc | 49,50 % | (1) |
| Kartoffelstärke | Solanium Tuberosum (Potato Starch) | 7,50 % | (2) |
| Magnesiumstearat | Magnesium Sterate | 2,50 % | (1) |

| | | | |
|---|---|---|---|
| Phase B | | | |
| Isopropylstearat | Isopropyl Stearate | 9,14 % | (3) |
| Cetylpalmitat | Cetyl Palmitate | 0,53 % | (1) |
| Ewalin 1751 | Petrolatum | 0,20 % | (4) |
| Parfümöl Elegance # 79228 D MF | Parfum | 0,20 % | (5) |
| Propyl-4-hydroxybenzoat | Propylparaben | 0,10 % | (1) |

Herstellung:

Bestandteile der Phase A zusammen geben und vormischen.
Anschließend die Pudermischung unter Rühren tropfenweise mit der geschmolzenen Phase B versetzen. Die Puder werden bei 40 - 50 bar gepresst.

Bezugsquellen:

(1) Merck KGaA
(2) Südstärke GmbH
(3) Cognis GmbH
(4) H. Erhard Wagner GmbH
(5) Haarmann & Reimer GmbH

**Beispiel G: Creme Mascara (O/W)**

Phase A

| | | | |
|---|---|---|---|
| Mica Black | CI 77499 (Iron Oxides), Mica, CI 77891 (Titanium Dioxide) | 10,00 % | (1) |
| Pigment | | 5,00 % | (1) |

| | | | |
|---|---|---|---|
| Phase B | | | |
| Stearinsäure | Stearic Acid | 8,00 % | (1) |
| Bienenwachs gebleicht | Cera Alba (Beeswax) | 6,00 % | (1) |
| Carnaubawachs 2442 L | Copernicia Cerifera (Carnauba Wax) | 4,00 % | (2) |
| Eutanol G | Octyldodecanol | 3,00 % | (3) |
| Arlacel 83 V | Sorbitan Sesquioleate | 2,00 % | (4) |
| Propyl-4-hydroxybenzoat | Propylparaben | 0,10 % | (1) |
| RonaCareTM Tocoherolacetat | Tocopheryl Acetate | 0,50 % | (1) |

| | | | |
|---|---|---|---|
| Phase C | | | |
| Wasser, demineralisiert | Aqua (Water) | 50,84 % | |
| Triethanolamin reinst | Triethanolamine | 2,30 % | (1) |
| Water Soluble Shellac SSB 63 | Shellac | 8,00 % | (5) |
| Methyl-4-hydroxybenzoat | Methylparaben | 0,25 % | (1) |
| RonaCareTM Biotin | Biotin | 0,01 % | (1) |

Herstellung:

Alle Bestandteile der Phase B zusammen bei ca. 80 °C aufschmelzen, rühren bis alles geschmolzen ist. Die Beugungsfarbmittel der Phase A einrühren. Shellac der Phase C im Wasser lösen, auf 75 °C erwärmen. Die restlichen Bestandteile der Phase C zugeben, lösen. Bei 75 °C langsam unter Rühren die Phase C zu Phase A/B geben, 2 min homogenisieren. Die Masse unter Rühren auf Raumtemperatur abkühlen.

Bezugsquellen:

(1) Merck KGaA/Rona®
(2) Kahl & Co.
(3) Cognis GmbH
(4) Uniqema
(5) Paroxite Ltd.

**Beispiel H: Nagellack**

| | | | |
|---|---|---|---|
| Phase A | | | |
| Pigment | | 2,00 % | (1) |
| Thixotrope Nagellack-Base 1348 | Toluene, Ethyl Acetate, Butyl Acetate, Nitrocellulose, Tosylamide/Formaldehyde Resin, Dibutyl Phthalate, Isopropyl Alcohol, Stearalkonium Hectorite, Camphor, Acrylates Copolymer, Benzophenone-1 | 98,00 % | (2) |
| Farbdispersion mit Nitrocellulose Lack (q.s.) | | | |

Herstellung:

Das erfindungsgemäße wird zusammen mit der Lackbase eingewogen, gut mit einem Spatel von Hand vermischt und anschließend 10 min bei 1000 Upm gerührt.

Bezugsquellen:

(1) Merck KGaA/Rona®
(2) International Lacquers S. A.

**Beispiel J: Shampoo**

| | | | |
|---|---|---|---|
| Phase A | | | |
| Pigment | | 0,05 % | (1) |
| Carbopol ETD 2020 | Acrylates/C10-30 Alkyl Acrylate Crosspolymer | 0,90 % | (2) |
| Wasser, demineralisiert | Aqua (Water) | 59,90 % | |

| | | | |
|---|---|---|---|
| Phase B | | | |
| Triethanolamin reinst | Triethanolamine | 0,90 % | (1) |
| Wasser, demineralisiert | Aqua (Water) | 10,00 % | |

| | | | |
|---|---|---|---|
| Phase C | | | |
| Plantacare 2000 UP | Decyl Glucoside | 20,00 % | (3) |
| Texapon ASV | Magnesium Oleth Sulfate, Sodium Oleth Sulfate, Magnesium Laureth-8 Sulfate, Sodium Laureth-8 Sulfate, Magnesium Laureth Sulfate, Sodium Laureth Sulfate | 8,00 % | (3) |
| Bronidox L | Propylene Glycol, 5-Bromo-5-Nitro-1,3 -Dioxane | 0,20 % | (3) |
| Parfümöl Everest 79658 SB | Parfum | 0,05 % | (4) |

Herstellung:

Für Phase A das erfindungsgemäße Pigment in das Wasser einrühren. Mit einigen Tropfen Citronensäure (10%ig) ansäuern um die Viskosität zu vermindern und das Carbopol unter Rühren langsam einstreuen. Nach vollständiger Lösung langsam Phase B zugeben. Nacheinander werden nun die Bestandteile der Phase C zugegeben.

Bezugsquellen:

(1) Merck KGaA
(2) BF Goodrich GmbH
(3) Cognis GmbH
(4) Haarmann & Reimer GmbH

**Beispiel K: Künstlicher Nagel mit Opaleffekt**

Ein Film wird entsprechend einem der Beispiele 7, 9, 11, 11a oder 14c ohne den beschriebenen Mahlschritt hergestellt und in eine Form gebracht die sich als künstlicher Nagel bzw. als Beschichtung für einen Nagel eignet.

**Patentansprüche**

1. Verwendung von Beugungsfarbmitteln in der Kosmetik.

2. Verwendung nach Anspruch 1, **dadurch gekennzeichnet, dass** die Beugungsfarbmittel zu dekorativen Zwecken in der topischen Anwendung eingesetzt werden.

3. Verwendung nach Anspruch 1, **dadurch gekennzeichnet, dass** die Beugungsfarbmittel zu dekorativen Zwecken in Zubereitungen zur topischen Anwendung eingesetzt werden.

4. Verwendung nach mindestens einem der vorstehenden Ansprüche, **dadurch gekennzeichnet, dass** es sich bei den Beugungsfarbmitteln um Partikel, Folien oder Aggregate handelt.

5. Verwendung nach mindestens einem der vorstehenden Ansprüche, **dadurch gekennzeichnet, dass** die Beugungsfarbmittel in der Nagelkosmetik eingesetzt werden.

**6.** Verwendung nach mindestens einem der vorstehenden Ansprüche, **dadurch gekennzeichnet, dass** die Beugungsfarbmittel in dem für die optischen Eigenschaften wesentlichen Anteil im wesentlichen aus Anordnungen kugelförmiger Teilchen oder Kavitäten mit einer im wesentlichen monodispersen Größenverteilung bestehen.

**7.** Verwendung nach Anspruch 6, **dadurch gekennzeichnet, dass** die kugelförmigen Teilchen in eine wachsartige Matrix eingebettet sind.

**8.** Verwendung nach mindestens einem der vorstehenden Ansprüche, **dadurch gekennzeichnet, dass** die kugelförmigen Teilchen im wesentlichen aus einem organischen Polymer, das vorzugsweise vernetzt ist, bestehen.

**9.** Verwendung nach mindestens einem der Ansprüche 1 bis 7, **dadurch gekennzeichnet, dass** die kugelförmigen Teilchen im wesentlichen aus einem anorganischen Material, vorzugsweise einem Metall oder Halbmetall oder einem Metallchalcogenid oder Metallpnictid bestehen, wobei insbesondere Silciumdioxid, Aluminiumoxid, Zirkonoxid, Eisenoxide, Titandioxid, Cerdioxid, Galliumnitrid, Bor- und Aluminiumnitrid sowie Silicium- und Phosphornitrid oder Mischungen davon als Kernmaterial bevorzugt sind.

**10.** Verwendung nach mindestens einem der Ansprüche 1 bis 7, **dadurch gekennzeichnet, dass** die kugelförmigen Teilchen im wesentlichen aus Polymeren bestehen, die Partikel, beispielsweise Metalloxide, eingeschlossen enthalten.

**11.** Verwendung nach mindestens einem der vorstehenden Ansprüche, **dadurch gekennzeichnet, dass** die Beugungsfarbmittel in dem für die optischen Eigenschaften wesentlichen Anteil im wesentlichen aus Kern-Mantel-Partikeln bestehen, deren Mantel eine Matrix bildet und deren Kern im wesentlichen fest ist und eine im wesentlichen monodisperse Größenverteilung aufweist, wobei ein Unterschied zwischen den Brechungsindices des Kernmaterials und des Mantelmaterials besteht.

**12.** Verwendung nach Anspruch 11, **dadurch gekennzeichnet, dass** die Matrix spröde ist und es sich bei dem Mantelmaterial vorzugsweise um ein homo- oder copolymeres Poly(cyclohexylmethacrylat), Polystyrol sowie substituierte Polystyrolderivate, wie z. B. Poly(iodstyrol) und Poly(bromstyrol), Polyacrylate und Polymethacrylate mit einer Tg oberhalb der Gebrauchstemperatur, Polyvinylchlorid mit hoher Tg und andere vinylische Polymere, die sich durch Umwandlung aus Polyvinylacetat ergeben, Polyacrylnitril und Styrol-Acrylnitril-Copolymere handelt.

**13.** Verwendung nach mindestens einem der Ansprüche 1 bis 6, **dadurch gekennzeichnet, dass** die Beugungsfarbmittel Kavitäten aufweisen und die Matrix um die Kavitäten im wesentlichen aus einem organischen Polymer, das vorzugsweise vernetzt ist, besteht.

**14.** Verwendung nach mindestens einem der Ansprüche 1 bis 6, **dadurch gekennzeichnet, dass** die Beugungsfarbmittel Kavitäten aufweisen und die Matrix um die Kavitäten im wesentlichen aus einem anorganischen Material, vorzugsweise einem Metall oder Halbmetall oder einem Metallchalcogenid oder Metallpnictid bestehen, wobei insbesondere Silciumdioxid, Aluminiumoxid, Zirkonoxid, Eisenoxide, Titandioxid, Cerdioxid, Galliumnitrid, Bor- und Aluminiumnitrid sowie Silicium- und Phosphornitrid oder Mischungen davon bevorzugt sind.

**15.** Verwendung nach mindestens einem der Ansprüche 1 bis 6 oder 13, **dadurch gekennzeichnet, dass** die Matrix thermoplastische oder duroplastische Eigenschaften aufweist und vorzugsweise aus Poly(styrol), thermoplastischen Poly(acrylat)-derivaten, vorzugsweise Poly(methylmethacrylat) oder Poly(cyclohexylmethacrylat), bzw. thermoplastischen Copolymeren dieser Polymere mit anderen Acrylaten, wie vorzugsweise Styrol-Acrylnitril-Copolymeren, Styrol-Ethylacrylat-Copolymeren oder Methylmethacrylat-Ethylacrylat)-Copolymeren aufgebaut ist.

**16.** Verwendung nach mindestens einem der Ansprüche 1 bis 6, **dadurch gekennzeichnet, dass** die Beugungsfarbmittel Kavitäten aufweisen und die Matrix eine Carbon-Matrix ist.

**17.** Verwendung nach mindestens einem der vorstehenden Ansprüche,
**dadurch gekennzeichnet, dass** der mittlere Durchmesser der kugelförmigen Partikel oder Kavitäten in den Beugungsfarbmitteln im Bereich von etwa 50 - 500 nm, bevorzugt im Bereich von 100 - 500 nm und ganz besonders bevorzugt im Bereich von 200 bis 280 nm liegt.

**18.** Zubereitung zur topischen Anwendung enthaltend mindestens ein Beugungsfarbmittel und mindestens einen zur topischen Anwendung geeigneten Träger.

**19.** Zubereitung nach Anspruch 18, **dadurch gekennzeichnet, dass** die Gesamtmenge des oder der Beugungsfarbmittel aus dem Bereich von 0,1 Gew.-% bis 30 Gew.-%, bezogen auf das Gesamtgewicht der Zubereitung, gewählt wird.

**20.** Zubereitung nach mindestens einem der vorstehenden Ansprüche **dadurch gekennzeichnet, dass** die Zubereitung mindestens einen Farbstoff oder ein Pigment enthält, wobei die Zubereitung vorzugsweise mindestens ein Effektpigment, insbesondere ein Perlglanzpigment enthält.

**21.** Zubereitung nach mindestens einem der vorhergehenden Ansprüche,
**dadurch gekennzeichnet, dass** die Zubereitung mindestens eine Substanz enthält, die UV-Strahlung im UV-A- und/oder UV-B-Bereich absorbiert, wobei die mindestens eine Substanz vorzugsweise ausgewählt ist aus 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-diphenyl-acrylsäure-2-ethylhexylester, 2-Phenyl-benzimidazol-5-sulfonsäure sowie deren Kalium-, Natrium- und Triethanol-aminsalzen.

**22.** Verfahren zur Herstellung einer Zubereitung zur topischen Anwendung,
**dadurch gekennzeichnet, dass** mindestens ein Beugungsfarbmittel mit mindestens einem zur topischen Anwendung geeigneten Träger und gegebenenfalls weiteren Inhaltsstoffen vermischt wird.

**23.** Verfahren zur Beurteilung der Auftragung von Zubereitungen zur topischen Anwendung, **dadurch gekennzeichnet, dass** man Beugungsfarbmittel, welche in die Zubereitung eingearbeitet sind, auf die Haut aufträgt und anhand des sichtbaren Effekts der Beugungsfarbmittel abschätzt, auf welchen Hautpartien bereits ein Auftrag stattgefunden hat und/oder ob genügend Zubereitung aufgetragen worden ist und/oder wann ein erneutes Auftragen erforderlich geworden ist.

**24.** Beugungsfarbmittel, **dadurch gekennzeichnet, dass** es sich um ein Aggregat aus Anordnungen kugelförmiger Teilchen mit einer im wesentlichen monodispersen Größenverteilung in einer wachsartigen Matrix handelt.

**25.** Beugungsfarbmittel nach Anspruch 24, **dadurch gekennzeichnet, dass** die wachsartige Matrix gewählt ist aus Fetten, Wachsen und anderen natürlichen und synthetischen Fettkörpern, vorzugsweise Estern von Fettsäuren mit Alkoholen niedriger C-Zahl, z.B. mit Isopropanol, Propylenglykol oder Glycerin, oder Estern von Fettalkoholen mit Alkansäuren niedriger C-Zahl oder mit Fettsäuren, wobei die wachsartige Matrix vorzugsweise gewählt ist aus Cetylpalmitat und Lanolin.

**26.** Verfahren zu deren Herstellung eines Beugungsfarbmittels, wobei in einem ersten Schritt eine dreidimensionale Kugelpackung erzeugt wird und in einem zweiten Schritt die Kugelaggregate mit einer Schmelze eines bei Raumtemperatur wachsartigen Materials getränkt werden.

**27.** Künstlicher Nagel enthaltend Beugungsfarbmittel.

**28.** Nagelpflegemittel enthaltend Lösungsmittel und Bindemittel, **dadurch gekennzeichnet, dass** das Nagelpflegemittel Beugungsfarbmittel oder Vorstufen für Beugungsfarbmittel, insbesondere in Form monodisperser Kugeln oder von Kern-Mantel-Partikeln, enthält.

**29.** Nagelpflegemittel nach Anspruch 28, **dadurch gekennzeichnet, dass** es sich um einen Nagellack auf Wasserbasis handelt, der vorzugsweise Polyurethane oder Polyacrylate als Bindemittel enthält.

**30.** Nagelpflegemittel nach mindestens einem der Ansprüche 28 oder 29, **dadurch gekennzeichnet, dass** das Beugungsfarbmittel oder die Vorstufe für das Beugungsfarbmittel gleichzeitig als Bindemittel dient, wobei das Nagelpflegemittel vorzugsweise kein weiteres Bindemittel enthält und es sich bei der Vorstufe für das Beugungsfarbmittel vorzugsweise um Kern-Mantel-Partikel, vorzugsweise mit Polyacrylat-Mantel handelt.

Europäisches Patentamt

# EUROPÄISCHER RECHERCHENBERICHT

Nummer der Anmeldung
EP 05 01 3458

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 01/88044 A (MERCK PATENT GMBH; ANSELMANN, RALF; ALBRECHT, THOMAS; RODRIGUEZ-MOZAZ,) 22. November 2001 (2001-11-22)<br>* Seite 2, Zeile 20 - Zeile 27 *<br>* Seite 3, Zeile 8 - Zeile 16 *<br>* Seite 3, Zeile 18 - Seite 7, Zeile 16 *<br>* Seite 12, Zeile 16 - Zeile 20 *<br>* Beispiele *<br>* Ansprüche *<br>----- | 1-9, 17-20, 22,27-30 | A61Q1/02<br>A61Q1/12<br>A61Q3/02<br>A61K8/04<br>A61K8/81<br>A61K8/25<br>A61K8/92 |
| D,X | WO 03/025035 A (MERCK PATENT GMBH; ANSELMANN, RALF; WINKLER, HOLGER; HELLMANN, GOETZ,) 27. März 2003 (2003-03-27)<br>* Seite 31, Absatz 4 *<br>* Beispiele *<br>* Ansprüche *<br>----- | 1-12,15, 17-20, 22,27-30 | |
| X | US 2002/024163 A1 (FU GUOYI ET AL) 28. Februar 2002 (2002-02-28)<br>* Absatz [0025]; Ansprüche 1,7; Beispiele *<br>----- | 1-15, 17-20, 22,27-30 | RECHERCHIERTE SACHGEBIETE (Int.Cl.7)<br>A61K |
| A | EP 0 615 748 A (HEINZE, FRIEDRICH, DR) 21. September 1994 (1994-09-21)<br>----- | | |

Der vorliegende Recherchenbericht wurde für alle Patentansprüche erstellt

2

| Recherchenort | Abschlußdatum der Recherche | Prüfer |
|---|---|---|
| München | 10. Oktober 2005 | Pregetter, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 05 01 3458

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-10-2005

| Im Recherchenbericht angeführtes Patentdokument | | Datum der Veröffentlichung | Mitglied(er) der Patentfamilie | | | Datum der Veröffentlichung |
|---|---|---|---|---|---|---|
| WO 0188044 | A | 22-11-2001 | AU | 5632001 | A | 26-11-2001 |
| | | | CA | 2408990 | A1 | 15-11-2002 |
| | | | CN | 1443223 | A | 17-09-2003 |
| | | | DE | 10024466 | A1 | 22-11-2001 |
| | | | EP | 1285031 | A1 | 26-02-2003 |
| | | | JP | 2003533579 | T | 11-11-2003 |
| | | | US | 2003116062 | A1 | 26-06-2003 |
| WO 03025035 | A | 27-03-2003 | BR | 0212478 | A | 24-08-2004 |
| | | | CA | 2459749 | A1 | 27-03-2003 |
| | | | CN | 1553925 | A | 08-12-2004 |
| | | | EP | 1425322 | A2 | 09-06-2004 |
| | | | JP | 2005503460 | T | 03-02-2005 |
| | | | MX | PA04002266 | A | 29-06-2004 |
| | | | US | 2004253443 | A1 | 16-12-2004 |
| US 2002024163 | A1 | 28-02-2002 | US | 2004262790 | A1 | 30-12-2004 |
| EP 0615748 | A | 21-09-1994 | CN | 1095953 | A | 07-12-1994 |
| | | | DE | 4308282 | A1 | 22-09-1994 |
| | | | JP | 7002644 | A | 06-01-1995 |
| | | | US | 5496565 | A | 05-03-1996 |

EPO FORM P0461

Für nähere Einzelheiten zu diesem Anhang : siehe Amtsblatt des Europäischen Patentamts, Nr.12/82